# EUROPEAN PATENT APPLICATION

(11) **EP 1 512 743 A1**
(43) Date of publication of application: **09.03.2005**
(21) Application number: 04104331.6
(22) Date of filing: 08.09.2004
(51) Int. Cl.: C12N 15/11, C12N 15/54, C12N 15/79, C12N 5/10, A61K 48/00

(54) **Conditional modification of siRNAs expression**

(30) Priority: 08.09.2003 EP 03020267
(71) Applicant: Odenthal, Margarete, 50937 Köln (DE); Breuhahn, Kai, Dr., 50858 Köln (DE); Fries, Jochen W. U., Dr., 50259 Pulheim (DE)
(72) Inventor: Odenthal, Margarete, 50937 Köln (DE); Breuhahn, Kai, Dr., 50858 Köln (DE); Fries, Jochen W. U., Dr., 50259 Pulheim (DE)
(74) Representative: Helbing, Jörg, Dr.

(57) **Abstract**

The present invention provides an *in vitro* and *in vivo* method for modulating the expression of a target gene in a conditional manner, utilizing small interfering (si) RNA. It also provides non-eukaryotic RNA polymerase expression constructs and siRNA expression constructs suitable for said method, a kit comprising said expression cassettes as well as the use of said expression cassettes for the preparation of medicaments for the treatment of various diseases associated with dysfunctional gene expression.

By functionally fusing the nucleotide sequence of a non-eukaryotic-RNA-polymerase (*N-EU-Pol*) with eukaryotic transcription regulating sequences, an induced cell-type specific or otherwise regulated expression of the foreign RNA polymerase in eukaryots becomes possible. Since the non-eukaryotic-RNA polymerase lacks a nucleic signal peptide, it will remain in the cytoplasm after translation. Thus, it is only able to transcribe target sequences cytoplasmatically, and the nucleus-bound functions such as the addition of a cap-structure or of a polyA-tail will not take place. siRNA coding sequences, which are functionally linked with a recognition sequence for the non-eukaryotic-RNA-polymerase will be transcribed in the cytoplasm by the non-eukaryotic-RNA-polymerase, resulting in non-capped and non- polyadenylated siRNAs. Since the expression of the non-eukaryotic-RNA-polymerase cell-type specific or otherwise regulated, the expression of the siRNA is accordingly also cell-type specific or otherwise regulated. The functional link between coding sequences for siRNA with a recognition sequence for a conditionally regulated and expressed non-eukaryotic foreign-RNA polymerase will finally result in an intracellularly located, conditionally directed synthesis and function of siRNA in higher eukaryotic cells.

## Description

The present invention provides an *in vitro* and *in vivo* method for modulating the expression of a target gene in a conditional manner, utilizing small interfering (si) RNA. It also provides non-eukaryotic polymerase expression constructs and siRNA expression constructs suitable for said method, a kit comprising said expression constructs as well as the use of said expression constructs for the preparation of medicaments for the treatment of various diseases associated with dysfunctional gene expression.

By functionally fusing the nucleotide sequence of a non-eukaryotic-RNA-polymerase with eukaryotic transcription regulating sequences, an induced cell-type specific or otherwise regulated expression of the foreign RNA polymerase in higher eukaryots, viz. animals and plants, becomes possible. Since the non-eukaryotic-RNA polymerase lacks a nucleic signal peptide, it will remain in the cytoplasm after translation. Thus, it is only able to transcribe target sequences cytoplasmatically, and the nucleus-bound functions such as the addition of a cap-structure or of a polyA-tail will not take place. siRNA coding sequences, which are functionally linked with a recognition sequence for the non-eukaryotic-RNA-polymerase will be transcribed in the cytoplasm by the non-eukaryotic-RNA-polymerase, resulting in non-capped and non- polyadenylated siRNAs. Since the expression of the non-eukaryotic-RNA-polymerase cell-type specific or otherwise regulated, the expression of the siRNA is accordingly also cell-type specific or otherwise regulated. The functional link between coding sequences for siRNA with a recognition sequence for a conditionally regulated and expressed non-eukaryotic foreign-RNA polymerase will finally result in an intracellularly bcated, conditionally directed synthesis and function of siRNA in higher eukaryotic cells.

### Background of the Invention

In several human diseases, for example cancer, impaired wound healing, infectious-, metabolic-, chronic- and inflammatory diseases, etc., gene expression frequently becomes dysfunctional and/or proteins accumulate mutations and become inactive.

Molecular techniques such as the inhibition of a dysfunctional or mutated genes via oligonucleotides have failed to achieve clinical relevance because of their low efficiency and/or their high costs. However, a new technique called RNA interference (RNAi), whereby small RNA molecules defined as small interfering RNAs (siRNAs) interact in a sequence specific manner with cellular RNAs and thereby induce their degradation, has become a promising, powerful and widely used tool in laboratories for modulating gene function in invertebrates, mammals and plants (Vaucheret et al., *J. Cell Sci.,* 114, 3083-91 (2001); McManus and Sharp, *Nat. Rev. Genet.,* 3, 737-47 (2002); Sharp P.A., *Genes Dev.,* 15, 485-90 (2001)). This technique also appears to have great potential for clinical applications.

RNAi is an evolutionary conserved mechanism that generates the active siRNAs through an endogenous RNase (Dicer) activity in a multistep process. Subsequently, the resulting 19- to 23-nt siRNAs serve as guides for enzymatic cleavage of complementary homologous target RNAs; thus RNAi provides a mechanism to reduce expression of a target RNA (Elbashir et al., *Nature,* 411, 494-8 (2001); Elbashir et al., *Embo. J.,* 20, 6877-88 (2001); Hammond et al., *Nature,* 404, 293-6 (2000); Papp et al., *Plant Physiol.,* 132, 1382-90 (2003)). Double stranded RNAs (dsRNAs) longer than 30 nt, on the other hand, do activate an antiviral interferon response in mammals, leading to the unspecific degradation of RNA and consequently to an overall reduced protein biosynthesis and inhib ition of cell proliferation; thus long dsRNAs do not cause RNAi. However, in mammals smaller *in vitro* generated dsRNAs (21 nt in length) have been successfully used for specifically inhibiting the gene expression without inducing the interferon response of the host and thus they also act as siRNAs (Elbashir et al., *Nature,* 411, 494-498 (2001); Elbashir et al., *Genes Dev.,* 15, 188-200 (2001); Leirdal and Sioud, *Biochem. Biophys. Res. Commun.*, 295, 744-8 (2002)). Moreover, the artificially provided siRNAs and natural siRNAs are amplified by endogenous RNA-dependent RNA polymerases, intensifying the effects of the initial siRNA molecules (Miyagishi and Taira, *Nat. Biotechnol.,* 20, 497-500 (2002); Lipardi et al., *Cell,* 107, 297-307 (2001)). The natural roles of siRNA includes the defense against viral infection (Sharp P.A., *Genes Dev.,* 15, 485-90 (2001); Li W.X. and Ding S.W., *Curr. Opin. Biotechnol.,* 12, 150-4 (2001); Lucy et al., *Embo. J.,* 19, 1672-80 (2000); Li et al., *Embo. J.*, 18, 2683-91 (1999)) and the regulation of the expression of cellular genes (Elbashir et al., *Nature,* 411 , 494-8.28 (2001); Aravin et al., *Curr. Biol.,* 11 ,101 7-27 (2001 )) .

It has already been established with animal models that siRNA is effective in combating viral diseases through hydrolysis of the viral RNA, thus making it a potential therapeutic tool (McCaffrey et al., *Nature,* 418, 38-9 (2002); Lee et al., *Nat. Biotechnol.,* 20, 500-5 (2002); Novina et al., *Nat. Med.,* 8, 681-6 (2002); Carmichael G.G., *Nature,* 418, 379-80 (2002); Coburn and Cullen, *J. Virol.,* 76, 9225-31 (2002); Jiang M. and Milner J., *Oncogene,* 21 , 6041 -8 (2002); Olson et al., *Insect Biochem. Mol. Biol.,* 32, 1333-43 (2002); Capodici et al., *J. Immunol.,* 169, 5196-201 (2002); Kitabwalla M., Ruprecht R.M., *N. Engl. J. Med.,* 347, 1364-7 (2002); Randall et al., *Proc. Natl. Acad. Sci. USA,* 100, 235-40 (2003); Couzin J., *Science,* 299, 995 (2003); Wilson et al., *Proc. Natl. Acad. Sci. USA,* 100, 2783-8 (2003)).
For chronic, inflammatory-dependent, metabolic or tumorigenic diseases etc., or respective animal models no such basis exist so far and putative siRNA therapies for these have not been yet attempted. The reason for this limited application is the present lack of a suitable directional control of the *in vivo*-synthesis of siRNAs. A so-called drug targeting system is missing. In contrast to antiviral approaches, where the viral RNA serves as a target sequence for hydrolysis, approaches to suppress the synthesis of proteins belonging to the body itself, or to one of its organs, require a tightly controlled hydrolysis of target sequences. Therefore, in order to avoid lethal or toxic side effects, a regulated siRNA expression system is crucial to enable specific degradation of target sequences in a controlled manner, i.e. only in specific cell types of the organisms or under defined circumstances. In addition, conditionally driven siRNA synthesis and function provides a possibility for controlled manipulation of gene expression in animals and plants, in order to increase agricultural crop or to improve pathogen resistance. Furthermore, conditionally driven siRNA synthesis is an useful tool in basic research to study the function of a certain signaling pathways by silencing the corresponding genes during physiological and/or pathological situations *in vivo.*

Currently, siRNA is produced chemically or enzymatically through *in vitro* transcription using phage-RNA-polymerases (T7, Sp6 or T3) with consecutive transfection of these siRNA-molecules into the cells of interest (Elbashir et al., *Nature,* 411 , 494-8 (2001); Elbashir et al., *Genes Dev.,* 15, 188-200 (2001); Harborth et al., *J. Cell. Sci.,* 114, 4557-65 (2001); Elbashir et al., *Methods,* 26, 199-213 (2002); Krichevsky A.M. and Kosik K.S., *Proc. Natl. Acad. Sci. USA,* 99, 11926-9 (2002); Yang et al., *Mol. Cell. Biol.,* 21, 7807-16 (2001); Billy et al., *Proc. Natl. Acad. Sci. USA,* 98, 14428-33 (2001); Yang et al., *Proc. Natl. Acad. Sci. USA,* 99, 9942-7 (2002); DE-C-101 00 586; WO 00/44895, WO 02/44321). With these approaches, a transient effect of siRNA has been observed in cell culture systems. A more stable effect of siRNA in cells has been achieved using RNA-polymerase III for synthesis. After transfection of an expression cassette containing a siRNA-coding sequence under the control of a RNA polymerase III promoter, such as the U6 snRNA- or Pol-III H1 RNA promoter, the endogenous RNA-polymerase III mediated the transcription of the siRNA-coding sequence (Miyagishi and Taira K., *Nat. Biotechnol.,* 20, 497-500.35 (2002); Brummelkamp et al., *Science,* 296, 550-3 (2002); Paul et al., *Nat. Biotechnol.,* 20, 505-8 (2002); Calegari et al., *Proc. Natl. Acad. Sci. USA,* 99, 14236-40 (2002); Barton and Medzhitov, *Proc. Natl. Acad. Sci. USA,* 99, 14943-5 (2002); Shen et al., *EMBO Rep.,* 4, 609-15 (2003); Xia et al., *Nat. Biotechnol.,* 20, 1006-10 (2002); (WO 03/006477). Combining such a siRNA-coding expression cassette driven by endogenous RNA polymerases with a viral vector allows for stable and specific manipulation of gene expression through the siRNA, even in cells normally hard to transfect (Shen et al., *FEBS Lett.,* 539, 111-4.41 (2003); Xia et al., *Nat. Biotechnol.,* 20, 1006-10 (2002); Devroe and Silver, *BMC Biotechnol.,* 2, 15 (2002); Abbas-Terki et al., *Hum. Gene Ther.,* 13, 2197-201 (2002)).

The siRNA-coding sequence inserted in such expression cassettes of plasmids or viral vectors comprises two sequences, one representing the targeted RNA sequence (sense strand) and as a second sequence, a reverse and complementary one, coding for the strand hybridising to the targeted RNA (antisense strand). The antisense and sense strand can be located in different cassettes and expressed separately, subsequently hybridising to form a doublestranded RNA (dsRNA), or they can be located in one cassette and be synthesized as one molecule on a connected nucleic acid strand, provided a non-self-complementary sequence region (loop) is placed in between sense and antisense strand; an intramolecular stem-loop structure is then formed. So far by choice, the *in-vivo* synthesis of siRNAs in higher eukaryotic cells or organisms has been directed by regulatory sequences recognized by the eukaryotic RNA-polymerases III *(Pol III).* These polymerases have the advantage that they do not mediate the addition of a cap or a polyA-tail to the synthesized siRNAs, while at the same time they use an oligo-dT-sequence as termination signal. Most notably the eukaryotic H1 and U6 RNA promoters have been used for integration into siRNA expression cassettes, because they have a well-defined transcriptional start and mediate termination at a stretch of five thymidines, resulting in two base uridinylated overhangs, which is ideal for the expression of siRNA molecules. Another advantage of these promoters transcribed by *Pol III,* is the high copy number they yield. Based on these advantages, U6 and H1 promoters have already been used for ribozyme expression (Bertrand et al., *RNA,* 3, 75-88 (1997); Good et al., *Gene Ther.,* 4, 45-54 (1997); Thompson et al.; *Nucleic Acids Res.,* 23, 2259-68 (1995)) and now are most frequently used for siRNA expression (Miyagishi and Taira, *Nat. Biotechnol.,* 20, 497-500 (2002); Brummelkamp et al., *Science,* 296, 550-3 (2002); Paul et al., *Nat. Biotechnol.,* 20, 505-8 (2002); Barton and Medzhitov, *Proc. Natl. Acad. Sci. USA,* 99, 14943-5.42 (2002); Devroe and Silver, *BMC Biotechnol., 2,* 15 (2002); Hasuwa et al., *FEBS Lett.,* 532, 227-30 (2002); Dennis C., *Naturem* 420, 732 (2002)).

Since RNA-*Pol III* is ubiquitously and constitutively expressed in eukaryotic cells, efficient and stable expression of the siRNA-coding sequences contained in an expression cassette of a plasmid -, a viral or another vector, can be achieved by introducing these vectors into eukaryotic cells and/or the entire organisms. However, an unresolved problem of that approach is that the expression cannot be regulated. Present invention offers a solution to that drawback.

### Summary of the Invention

The present invention provides a method to modulate - *in vitro and in vivo -* the expression levels of target genes in a conditional manner by directing siRNA expression in higher eukaryotic cells, including human, animal and plant cells. The interventional modulation of the expression endogenous and/or exogenous genes becomes possible with the method of the present invention. Areas of application of the present invention are human health and basic research, where the ability to specifically modulate in a conditional manner the expression of genes, including homeostatic genes, offers great benefits. Further areas of application are genetics and agriculture, since animal and plant pathogens, or resistance mechanisms to these, are frequently responsible for sub-maximal yields and thus lower profits. The present invention allows the targeted modulation - in a tissue specific, a cell-type specific, species-specific or conditional manner - of genes responsible for these undesired effects, and therefore offers surprising benefits.

The present invention thus provides
(1) a method for the *in-vitro* modulation of the expression level of a target gene in higher eukaryotic cells or in higher eukaryotic tissue culture in a conditional manner which comprises
   (a) introducing into a higher eukaryotic cell or into a higher eukaryotic tissue culture
      (i) a non-eukaryotic RNA polymerase (hereinafter shortly referred terms *"N-EU-Pol")* expression construct carrying a coding sequence for the non-eukaryotic RNA polymerase under control of one or more regulating sequences providing for the conditional expression of the non-eukaryotic RNA polymerase and its conditional occurrence in the cytoplasma, and
      (ii) a siRNA expression construct carrying one or more siRNA coding sequences under control of a recognition sequence for the non-eukaryotic RNA polymerase wherein the expressed siRNA is capable of interaction with homologous mRNA sequences of the gene to be modulated; and
   (b) a conditional synthesis of the non-eukaryotic RNA polymerase in the cytoplasm of higher eukaryotic cells followed by a non-eukaryotic polymerase driven expression of the siRNA.;
(2) a non-eukaryotic RNA polymerase expression construct as defined in (1) above;
(3) a siRNA expression construct as defined in (1) above;
(4) a kit or gene transfer agent comprising the non-eukaryotic RNA polymerase expression construct and the siRNA expression construct as defined in (1) above;
(5) the use of the non-eukaryotic RNA polymerase expression construct as defined in (1) above for preparing a medicament for the prevention and/or treatment/therapy of viral infections, malignancies, metabolic dysfunction, chronic and acute injury, and hereditary predispositions in higher eukaryotic organisms, preferably humans;
(6) the use of the siRNA expression constructs as defined in (1) above for preparing a medicament for the prevention and or treatment/therapy of viral infections, malignancies, metabolic dysfunction, chronic and acute injury, and hereditary predispositions in higher eukaryotic organisms, preferably humans; and
(7) a method for the *in vivo* and *in vitro* modulation of the expression level of a target gene in higher eukaryotic cells, higher eukaryotic tissue culture or in the corresponding organisms in a conditional manner, which method comprises introducing into a higher eukaryotic cell or into a cell of the higher eukaryotic tissue culture or the corresponding organisms a non-eukaryotic RNA polymerase expression construct as defined in (1) above and a siRNA expression construct as defined in (1) above.

### Description of the Figures

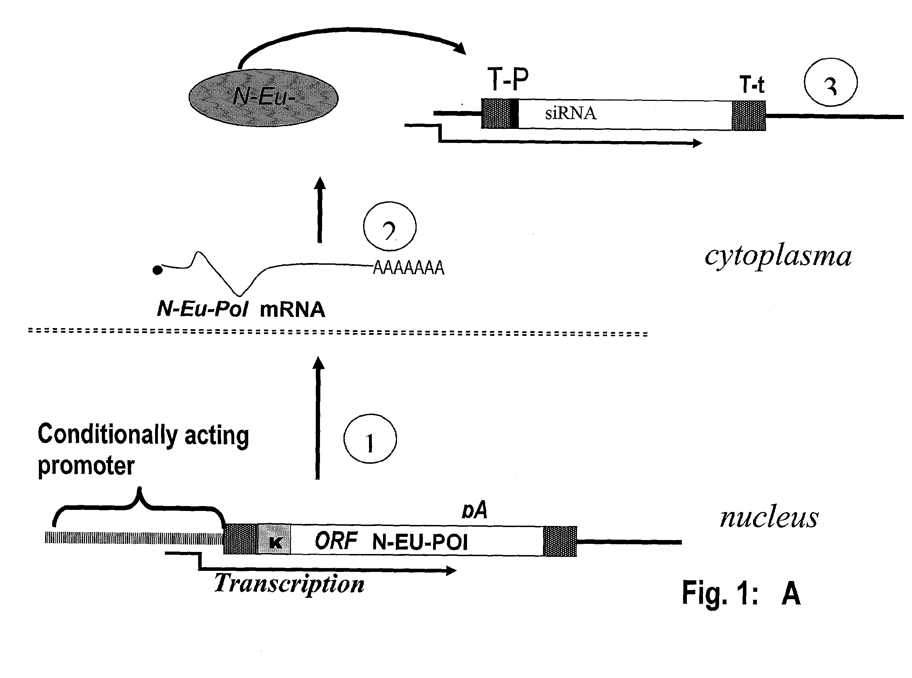

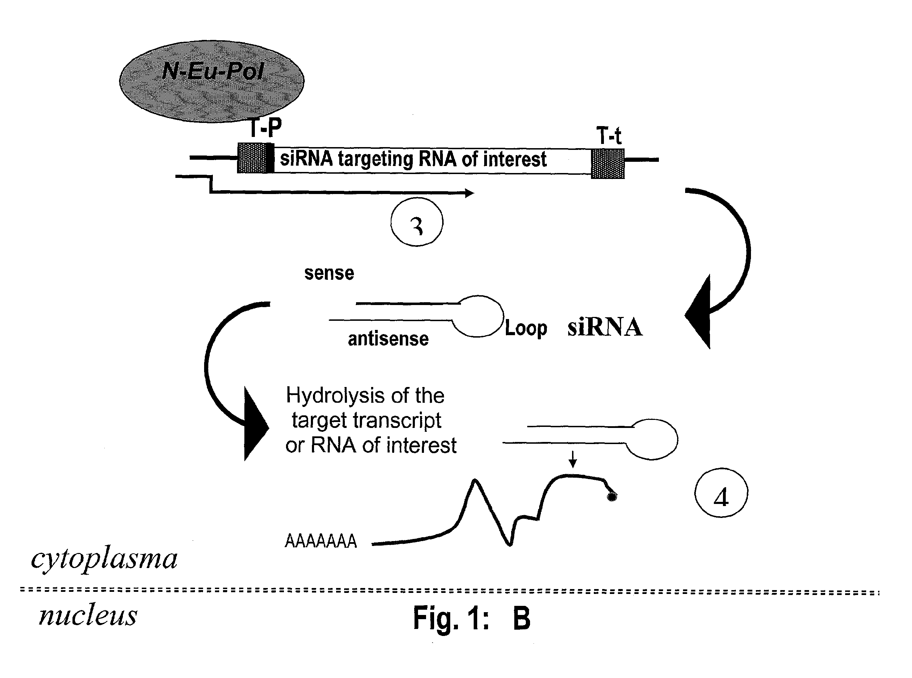

Figure 1 : Scheme of conditionally regulated siRNA function. A) The expression of non-eukaryotic RNA-polymerase *(N-EU-Pol)* ORF is linked to expression domains especially to a conditionally acting 5'-promoter fragment which provides for cell-type specific or conditional dependent synthesis of the *N-EU-Pol,* a Kozak sequence (K) and a poly-adenylation signal (pA). By functionally combining the *N-EU-Pol* sequence with eukaryotic protein expression signals, *N-EU-Pol* mRNAs are (1) transferred into the cytoplasma, where they (2) bind to ribosomes and are translated into a functional *N-EU-Pol,* which can act on an siRNA expression construct comprising *N-EU-Pol* recognition elements (T-P: *N*-EU-Pol-promoter). B) The functional link of siRNA coding DNA sequences to the recognition site (T-P: *(N-EU-Pol* promoter) for the *N-EU-Pol* leads to an efficient N-EU-Pol-dependent and specific siRNA synthesis and termination at the polymerase terminator (T-t) in the cytoplasma (3). In the cytoplasma the small hairpin (sh) RNA acts as an effector for the hydrolysis of the targeted transcripts (4).

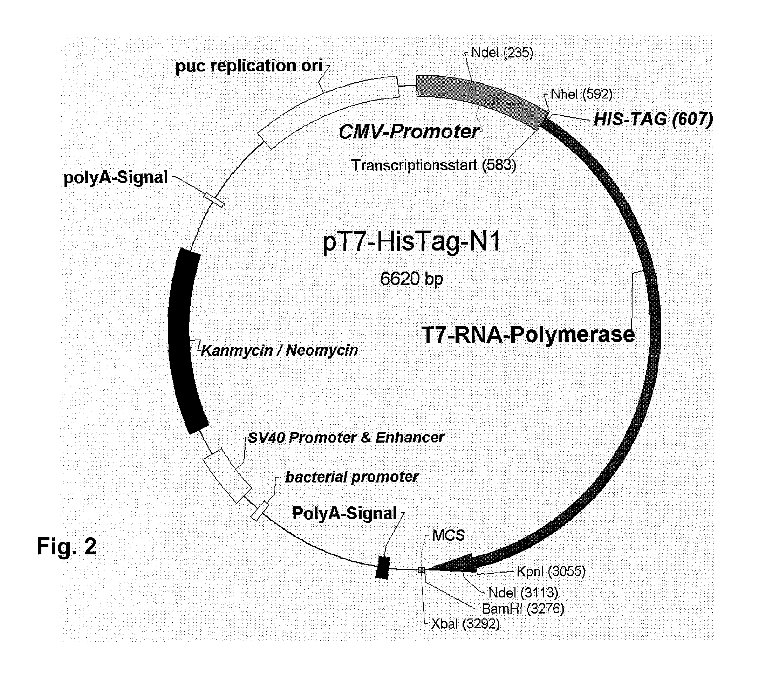

Figure 2 : Eukaryotic T7-phage RNA- Polymerase expression vector. The ORF of T7-phage RNA polymerase (SEQ I D NO: 1) has been cloned downstream to the CMV promoter/enhancer and upstream to the SV40 early m RNA polyadenylation signal of the plasmid backbone of pEGFP-C1 (BD Biosciences), in which the coding sequence of the Enhanced Green Fluorescent Protein gene (EGFP, SEQ I D NO: 36) had been deleted.

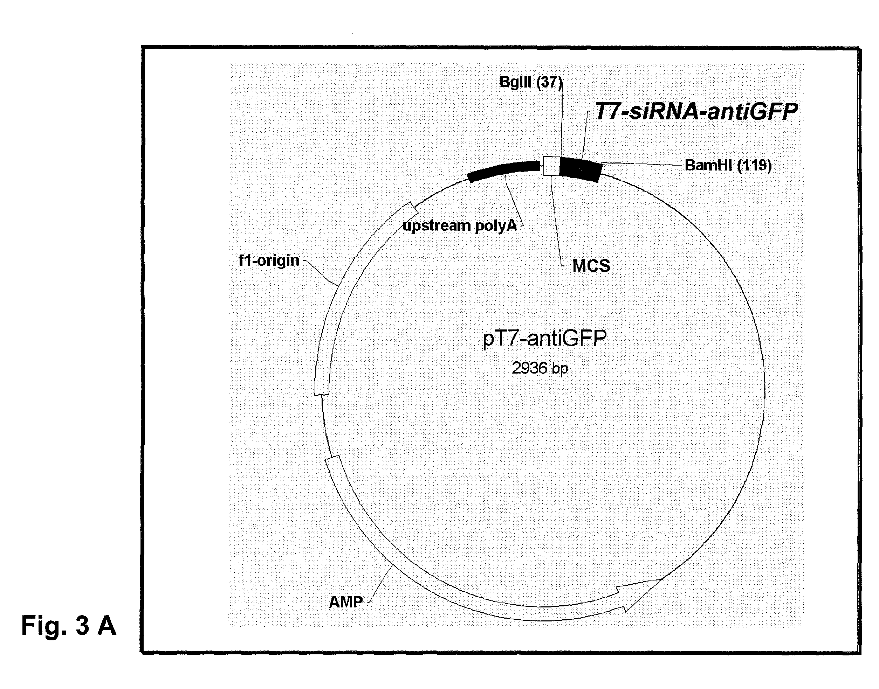

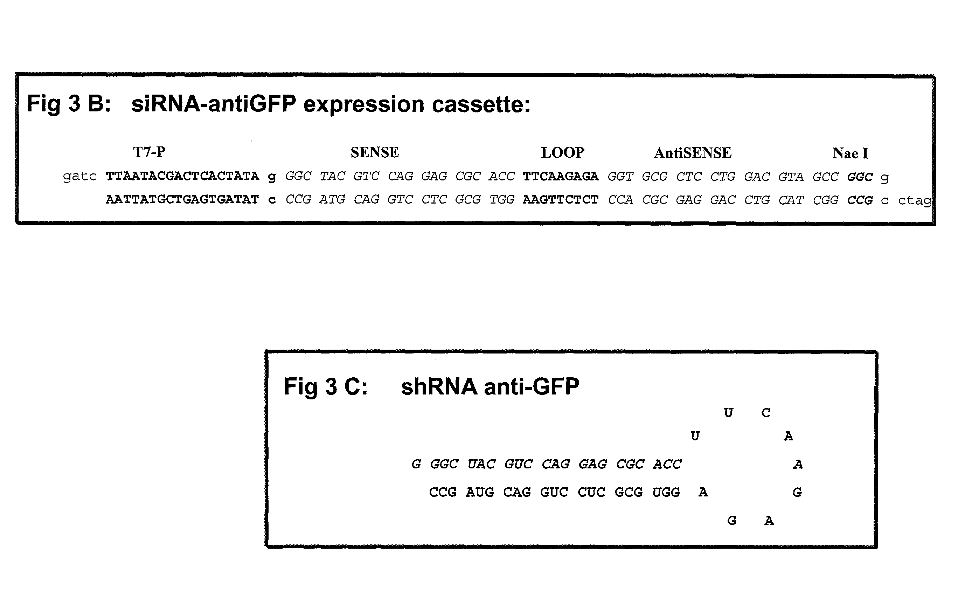

Figure 3: The anti-GFP siRNA expression system. A T7-phage RNA polymerase directed siRNA-antiGFP expression vector (pT7-Anti-GFP) was created. (A) The vector contains a Bgl II and BamH I flanked expression cassette including the promoter sequence of the gene 10 of the T7-entero-bacteriophage (T7-P) (SEQ ID NO: 7) and the coding sequence for siRNA targeting the EGFP transcripts (anti-GFP siRNA) (B). The anti-GFP siRNA coding sense and antisense coding strands are expressed as a small hairpin (sh)RNA (C) and both therefore located tandemly downstream of the T7-P, whereby the two sequences are separated by a loop region already described for the pSUPER vectors by Brummelkamp et al., *Science,* 296, 550-3 (2002). The beta-lactamase gene permits plasmid propagation in *E.coli.*

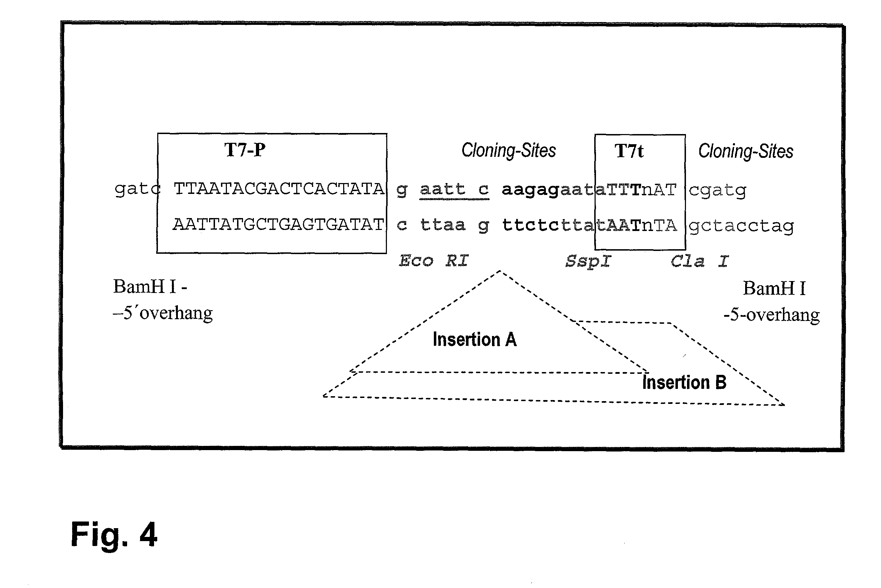

Figure 4: siRNA expression cassette with multiple insertion sites. An expression cassette is shown with a T7 promoter, a cloning site comprising Eco RI, Ssp and Cla I restriction sites allowing for two insertions A and B, and a T7 term inator. The cassette is carries at its 5' and 3' a Bam H I overhang.

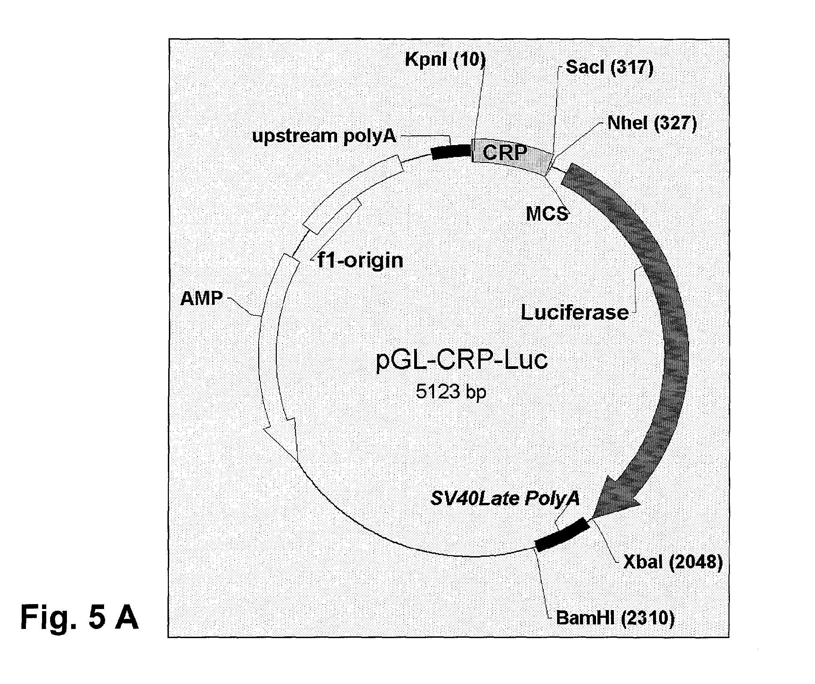

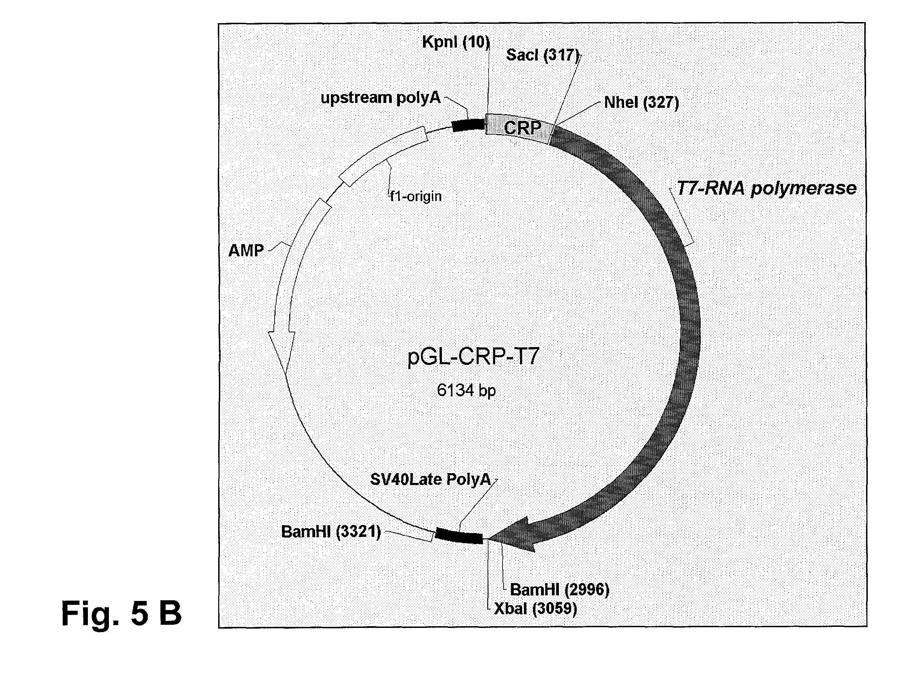

Figure 5: The conditional expression system of siRNA. Plasmid maps of the two plasmids (A) pGL-CRP-Luc and (B) pGL-CRP-T7.

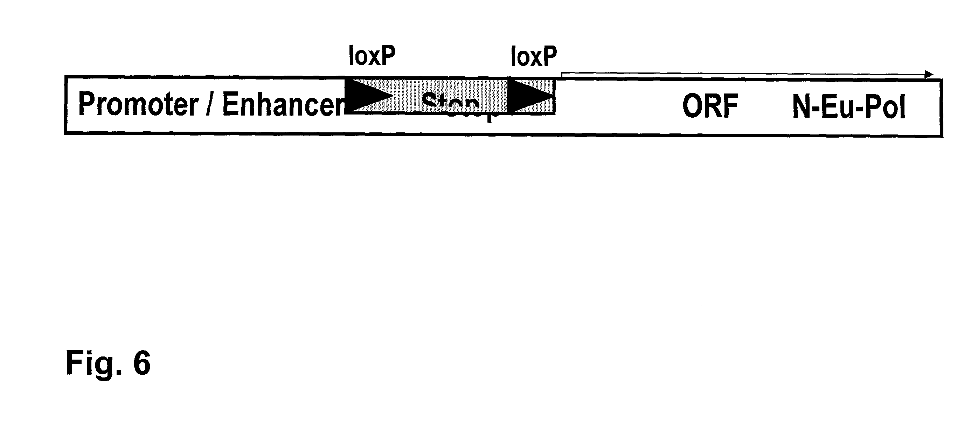

Figure 6: Conditionally regulated *N-EU-Pol* mediated by the CRE / LOX system. Scheme of an expression cassette for conditionally Cre-regulated synthesis of a N-*EU-Pol* resulting in conditionally controlled siRNA function. Expression of *N-EU-Pol* is im peded by a Westphal stop fragment (stop), which is flanked by IoxP sites. Conditional dependent cre-activity leads in excision of the stop fragment followed by *N-EU-Pol* expression which is controlled by a constitutive or inducible promoter of interest.

### Detailed Description of the invention

The presented invention describes a method, which enables the synthesis of siRNA in higher eukaryotic cells, preferably animal and plant cells, higher eukaryotic tissue culture or organism in a conditional, spatially and timewise directed manner, thereby allowing the modulation of expression level of a gene to be targeted by the siRNA.

"Higher eukaryotic organism" according to the present invention include animals such as vertebrates such as mammals (human and non-human, including rodents etc.), birds and fish; invertebrates such as worms and insects; plants, etc. "Higher eukaryotic cells" and "higher eukaryotic cell culture" according to the present invention relates to cells and cell culture derived from the above organisms, including stem cells and embryoid bodies.
A "higher eukaryotic tissue culture" according to the present invention relates to isolated tissue derived from the above organism, including intact organs.

*"In vitro"* and *"in vitro* cultivation" according to the present invention relates to the treatment of cells and tissue culture (including embryoid bodies and isolated human cells or organs). The above terms always refer to whole (functionally intact) cells and is not to be understood as referring to cellular extracts.

*"In vivo* and *"in vitro* cultivation" refer to the treatment of organisms or parts thereof not yet separated from the organism, i.e. treatment of cells and tissue in their natural environment.

"Modulation of the expression level" according to the invention relates to inhibition, and/or suppression of the target gene expression.

The conditional manner of modulating the expression in the method of embodiments (1) and (7) of the invention is effected by cell-, and/or tissue-, and/or development-specific factors, such as growth factors, hormones, cytokines or chemokines, receptors and/or pathophysiological factors, such as oxygen radicals, osmostic or oxidative stress and/or environmental factors such as temperature, ion concentration,and/or chemicals or drugs such as antibiotics, agonists an antagonists of channels, receptors, m ediators or the like.

In the method of embodiments (1) and (7) of the invention the expressed siRNAs interact with the homologous nucleic acid sequences of the gene to be modulated which results in a degradation of the homologous mRNA sequences and thus, in the inhibition and/or suppression of the gene encoding the homologous mRNA sequences.

Furthermore, it is preferred that the siRNAs are expressed in the cytoplasm and the siRNAs are void of a cap structure and a polyA tail. This can be achieved by the mode of administration of the siRNA expression construct and its design (i.e. by avoiding that the siRNA expression construct is present in the nucleus).
The expression construct according to the invention are circular or linear nucleic acids, preferably the expression constructs are vectors,, including plasmids, viruses, phages, most preferably expression constructs are adenoviral vectors. The *N-EU-Pol* expression construct may be designed to be integrated into the genome of the cells. The expression constructs are either extrachromosomal vectors or vectors with integration properties.

In the method of embodiments (1) and (7) of the invention the non-eukaryotic RNA polymerase is selected from a RNA polymerase derived from phages, viruses or bacteria. Preferably the non-eukaryotic RNA polymerase is selected from phage RNA polymerase such as a T7 RNA polymerase, a Sp6 RNA polymerase, a T3 RNA polymerase, T4 polymerase, a K11 RNA polymerase, a phiYeO3-12 RNA polymerase etc. In a particularly preferred embodiment of the invention the polymerase is a T7 RNA polymerase.

According to the present invention the regulating sequence providing for the conditional expression of the non-eukaryotic RNA polymerase is a genetic element controlling the cell- and/or tissue-, and/or development-specific, and/or pathophysiological and/or environmentally induced expression of the RNA polymerase, preferably the regulating sequence is selected from a cell and/or tissue and/or development-specific promoter, cell and/or tissue specific enhancers, and/or from regulatory elements of transcription mediating drugs, of reagents, of growth factors, of hormones or of the cytokine response. The regulating sequence may be combined with regulatory elements effecting the translation modus such as internal ribosome binding entry sites (I RES) or with specific splice sites resulting in conditional dependent splicing.
In a particular preferred embodiment of the invention the regulatory sequence is selected from a cell-type specific promoter and/ or enhancer such as the human epidermal cytokeratin 14 gene promoter or the liver-specific albumin promoter and /or the alpha-fetoprotein promoter and/or enhancer. According to the invention the regulatory sequences are also preferably selected from a pathophysiologically responsive promoter such as the liver-specific acute phase responsive promoter of the C-reactive protein gene and/or the respective enhancers and/or the respective control elements, the inflammatory inducible promoter and/or enhancer of the gene of the vascular cell adhesion molecule-1 or its hypoxic and/or cytokine responsive elements such as the NF?B binding sites. In a particular preferred embodiment regulatory sequences are selected from trancriptional control elements mediating a drug response such as the tetracycline responsive elements (TRE) of the bacterial tet operator (tetO).

According to the invention the non-eukaryotic RNA polymerase expression construct may comprise further genetic elements selected from constitutively active promoters, in particular of eukaryotic, prokaryotic or viral origin, recombinase recognition sites (RRS) such as lox P sites, attB/attP sites, stop codons, other bacterial control elements, internal ribosome entry sites (IRES), termination signals, polyA tail, mRNA stability elements, etc.

According to the invention the non-eukaryotic RNA polymerase is synthesized in the cytoplasm. For this purpose it is preferred that the expression constructs do not have sequences coding for nuclear signal peptides. The cytoplasmic localization of the non-eukaryotic RNA polymerase makes sure that neither cap structures nor polyA tails are added to the siRNAs which are transcribed by the respective non-eukaryotic RNA polymerase.

In the method of embodiments (1) or (7) of the invention the recognition sequences for the non-eukaryotic RNA polymerase of the siRNA expression construct are selected from a non-eukaryotic promoter such as bacteriophage promoters in particular RNA-polymerase binding elements taken from the T3, or T7 or the Sp6 bacteriophage genome and more preferably promoter sequences of the bacteriophage T7. It is preferred that said recognition sequences are functionally linked with sequences encoding the siRNA, and that said sequences coding for the siRNA are functionally linked with termination signals and/or hepatitis delta ribozyme encoding sequences and/or restriction sites.

According to the invention, the siRNAs encoded by the siRNA expression construct are individually designed to interact with the homologous sequences of the target gene to be modulated preferably, said siRNAs have length of 18 to 30 nucleotides, preferably of 25 to 30 nucleotides, more preferably of 18 to 25 nucleotides. The siRNA expression cassette may comprise additional genetic elements, preferably selected from recombinase recognition sites such as lox P sites or attB/attP sites possibly flanking a synthetic sequence and/or from bacterial regulatory sequences such as the tetracycline operator, tetO.

In a further embodiment of the invention the expressed siRNA forms a short hairpin RNA (shRNA), with the stem being formed by the sense strand and the antisense strand, and an interconnecting loop is in between these two strands. The total length of the shRNA between 41 and 85 nucleotides, whereby the minimal length of the antisense and sense strands is 18 nucleotides and the maximal length is 30 nucleotides; the loop comprises between 5 to 25 nucleotides.

In the method of embodiments (1) or (7), the non-eukaryotic promoter is preferably a promoter of the T7 bacteriophage selected from SEQ ID NO: 7 or variants thereof ( (SEQ ID NO: 52, SEQ I D NO: 53 and SEQ I D NO: 54), or an Sp6 promoter selected from SEQ ID Nos 8, 46, 47, or 48, or an T3 promoter selected from SEQ I D NO: 9 or 49, or a K11 promoter selected from SEQ I D NO: 55 or a phiYe03-12 promoter selected from SEQ ID NO: 56. The promoter are always matched at the end of the expression cassette with the appropriate termination signals, i.e. a T7 promoter with a T7 terminator (SEQ ID NOs: 37, 38, 39 or 40), a T3 promoter with a T3 term inator (SEQ I D NOs: 63 or 64), an Sp6 promoter with an rrnB T1 or T2 SEQ ID Nos: 65 or 66, respectively Sp6 terminator, a phiYe03-12 promoter with a phiYe03-12 terminator (SEQ ID NOs: 61 or 62) and a K11 promoter with a K11 terminator.

In a preferred embodiment of the invention the termination signal has SEQ ID Nos. 37, 38, 39 or 40. In a particular preferred mode of the invention the siRNA expression construct carries downstream of the siRNA or the shRNA encoding sequence a sequence encoding for a hepatitis delta ribozyme (SEQ ID Nos: 57-60) in order to produce homogenous, well-defined, and exact 3' ends of the siRNA transcripts by the cis-cleaving ribozyme.

In a particular preferred mode of the invention the non-eukaryotic polymerase expression construct has SEQ ID NOs: 67 and/or the siRNA expression construct has SEQ ID NO: 68 is a complex and/or the siRNA expression cassette is accom plished by dimerization of SEQ I D Nos: 69 and 70 out of the SEQ I D Nos: 26 and 27.
The method of embodiment (7) of the invention includes *in vitro* and *in vivo* reaction schemes. Thus, said method also embraces the treatment of the diseases mentioned in (5) and (6) above, i.e. a gene therapy method, which comprises administering an eukaryotic organism including agricultural crops and animals, preferably a human being, a polymerase expression construct and/or siRNA expression construct as described herein before.

By the same token the expression constructs utilized in the medicaments (5) and (6) are gene therapy vectors, and the constructs of (2) and (3) above may be gene therapy vectors.

The production of the expression constructs is hereinafter described in more detail, which shall, however, not be construed as to limit the invention.

### In vivo synthesis of siRNA by N-EU-Pol in higher eukaryotic cell systems

Recently published data showed that the endogenous RNA polym erase III is ideal for synthesis of siRNA. In addition, we were able to demonstrate that non-eukaryotic RNA polymerases *(N-EU-Pol)* are also suitable for synthesis of siRNA *in vivo* in a higher eukaryotic organism or a higher cell system. Up to now *N-EU-Pol,* in particular *N-EU-Pol* derived from enterobacteriophages, have only been used for siRNA synthesis in protozoa such as *Trypanosoma brucei* (Wang et al. Journal of Biological Chemistry 275, 40174-40179 (2000); Shi et al., RNA 6, 1069-1076 (2000) and for *in vitro* synthesis under definded reaction conditions (Yang et al., *Mol. Cell. Biol.,* 21 , 7807-16 (2001); Billy et al., *Proc. Natl. Acad. Sci. USA,* 98, 14428-33 (2001); Yu et al., *Proc. Natl. Acad. Sci. USA,* 99, 6047-52 (2002); Donze O. and Picard D., *Nucleic Acids Res.,* 30, e46 (2002) ) . Since capping reagents were lacking in the *in vitro* synthesis reaction, the siRNA was incapable of ribosomal binding after transfection in target cells. The experiments (example 1 and 2) given to support the present invention delineate the requirements for a successful *in vivo* siRNA synthesis by *N-EU-Pol.* Essential are the ability of the *N-EU-Pol* to be expressed in the eukaryotic cells and/or the whole organism, and for it to be localized in the cytoplasm, as well as the functional fusion of the siRNA-coding sequence with a corresponding promoter for *N-EU-Pol.*
Earlier studies have shown, that phage-RNA-polymerases can be expressed in protozoa (Wirtz et al., Nucleic Acid Research 26, 4626-4634 (1998)) but also in mammalian and plant cells (Elroy-Stein et al., *Proc. Natl. Acad. Sci. USA,* 86, 6126-30 (1989); Elroy-Stein O and Moss B., *Proc. Natl. Acad. Sci. USA,* 87, 6743-7 (1990); Deuschle et al., *Proc. Natl. Acad. Sci. USA,* 86, 5400-4 (1989); Fuerst et al., *Proc. Natl. Acad. Sci. USA,* 83, 8122-6 (1986); Moss et al., *Nature,* 348, 91-2 (1990); Huemer et al., *J. Virol. Methods,* 85, 1-10 (200); Alexander et al., *J. Virol.,* 66, 2934-42 (1992); Deng J. and Wolff J.A., *Gene,* 143, 245-9 (1994); US 5,135,855; US 5,126,251 WO 00/42206), and accordingly strategies were developed to use phage-RNA polymerases for eukaryotic *in vivo* protein synthesis (US 5,135,855 and 5,126,251) in mammalian and plant cells.

This limitation had to be overcome in phage RNA-polymerases intended to be used for protein synthesis in mammalian cells to guarantee the needed ribosomal binding (Elroy-Stein et al., *Proc. Natl. Acad. Sci. USA,* 86, 6126-30 (1989); Elroy-Stein O and Moss B., *Proc. Natl. Acad. Sci. USA,* 87, 6743-7 (1990); Deuschle et al., *Proc. Natl. Acad. Sci. USA,* 86, 5400-4 (1989); Fuerst et al., *Proc. Natl. Acad. Sci. USA,* 83, 8122-6 (1986); Moss et al., *Nature,* 348, 91-2 (1990); Huemer et al., *J. Virol. Methods,* 85, 1-10 (2000); Alexander et al., *J. Virol.,* 66, 2934-42 (1992); Deng J. and Wolff J.A., *Gene,* 143, 245-9 (1994); US-Patent 5,135855, and 5,125,251). Up to now, studies using phage-RNA polymerase for recombinant gene expression, fused the ORF of the phage-RNA-polymerase with the coding sequence of a nuclear signal peptide, also called nuclear localization signal (NLS) (Dunn et al., *Gene* 1988, 68, 259-66), in order to direct the polymerase to the nucleus, where the phage-RNA polymerase was able to perform capping (Huemer et al., *J. Virol. Methods,* 85, 1-10 (2000); WO 00/42206). Alternatively, to avoid the need for a cap in the initiation of translation, viral IRES- (internal ribosome entry site) and UTR- (untranslated regions) sequences have been used to enable efficient ribosome binding and protein translation more efficient in the cytoplasm (US-Patent 5,135,855), when a phage-RNA polymerase without an NLS was used. Unlike existing approaches in which T7 RNA polymerase is used as a tool for recombinant protein synthesis (Yarvoi and Pederson, Gene 275, 73-81 (2001), Dunn et al, Gene 68, 259-266 (1988); Huemer et al, J. Virol. Methods 85, 1-10 (2000); WO 00/42206), it is crucial for the present invention that the phage-RNA polymerases localize to the cytoplasm where they can direct siRNA synthesis, without adding a cap or a polyA tail. Therefore in the present invention NLS sequences are omitted in the expression cassette of the *N-EU-Pol.*

In contrast to prokaryotes, the recombinant expression of *N-EU-Pol* in eukaryotic systems can occur in various cellular compartments, and the *N-EU-Pol* are found to be transcriptionally active in all of them. By recombinantly fusing a Pol II promoter and a polyadenylation signal to the *N-EU-Pol* coding sequence, the *N-EU-Pol* transcript made in the nucleus of a eukaryotic cell is transported into the cytoplasm and is translated therein (figure 1 A). The nascent *N-EU-Pol,* having no signal peptide directing it to any of the specialized compartments such as nucleus, mitochondrium or the chloroplast, remains in the cytoplasm. The present invention demonstrates that the cytoplasmic *N-EU-Pol* is able to transcribe siRNA coding sequences (Examples 1 to 3, see also figure 2A), generating functional siRNAs. In the cytoplasm, where the *N-EU-Pol* of the present invention is active, no capping-mechanism and polyadenylation occur.
A further advantage of the localization of the *N-EU-Pol* itself to the cytoplasm and thus the siRNA synthesis there, is that this is also the compartment whereto endogenous target transcripts are primarily located (see Fig. 1 B).
To synthesize siRNA with *N-EU-Pol,* the siRNA-coding sequence was functionally fused with the recognition- and binding sequence, i.e. the promoter, of the corresponding *N-EU-Pol.* In the experiments of present invention, RNA-polymerase of the enterobacterio-phage T7 was used for siRNA synthesis in eukaryotic cell systems. Therefore the binding sequence of the promoter region of gene 10 of the enterobacteriophage T7 (SEQ ID NO: 7) was employed. Alternatively, variants of this promoter can be placed 5' of the siRNA-coding sequence to construct an expression cassette (SEQ ID Nos: 52, 53 and 54). These can be derived from other genes of bacteriophages or the consensus of the native T7 promoter may be synthetically varied. Another option is to place the recognition sequences and their variants of the respectively expressed *N-EU-Pol* into the expression cassette. When the *N-EU-Pol* of the bacteriophage Sp6 is expressed in eukaryotic cells to drive the expression of siRNAs, the recognition-and binding sequence, i.e. the promoter, recognized by Sp6-RNA Polymerase have to be used. These *N-EU-Pol* binding sequences (SEQ I D NO: 6) can be of native or synthetic origin. This is also true for other phage-based expression systems such as T3, PhiYe03-12 and K11. If a viral *N-EU-Pol* is expressed recombinantly, a corresponding viral promoter sequence in the siRNA expression cassette has to be employed. Preferably, phage expression systems are used, since they only require a short recognition sequence (less than 30 bp) for the corresponding RNA-polymerase, which is highly specific, while of the specific recognition sequence only a few nucleotides are crucial (Diaz et al., *J. Mol. Biol.,* 229, 805-11 (1993); Lee S.S. and Kang C., *Biochem. Int.,* 26, 1-5 (1992)).

In the method of present invention, the coding sequence of the siRNA will be expressed as a short hairpin RNA (shRNA) from one gene comprising both the sense and the antisense strand, whereby the two sequences are separated from each other by a non-complementary loop region. An expression cassette for synthesis of shRNA is depicted in example 2.1 (figure 3B), in which siRNA against enhanced green fluorescent protein (EGFP, SEQ ID NO: 36) was expressed in hepatoma cells, using T7 phage RNA polymerase. However, synthesis of sense and antisense strand of siRNA is also possible using one expression construct comprising between two oppositly directing promoters the sense and antisense coding region, or two separate expression cassettes, one for the sense and the other for the antisense strand.

Transcription from the presented expression cassette does not have to be stopped via a termination signal, if the sequence ends downstream of the expression cassette. For example, no further termination is necessary, if the expression cassette is chemically produced as an oligonucleotide-dimer, which can be directly transfected into eukaryotic cells. Preferably, the siRNA-expression cassette is localized in a plasmid or viral vector. Expression cassettes of viral vectors contain a termination signal, which either corresponds to the native terminators of the individual *N-EU-Pol* (e.g. native T7-terminators, c.f. SEQ ID NOs: 37-40) or represents a more efficient variant or a synthetically designed terminator. The terminators are located downstream of the coding region of the siRNA strands. When siRNA is expressed as shRNA variants, a oligo-dT domain might be placed downstream of the hairpin siRNA sequence. When siRNA-expression cassettes localized in plasmids are used such as described in the examples, termination of siRNA synthesis is guaranteed by plasmid linearisation. Thus the siRNA expression cassette contains one or more restriction sites in the 3' siRNA-flanking region (figure 4) for linearisation. In order to produce exact and correctly terminated 3' ends of siRNA or shRNA, a sequence encoding for a hepatitis delta virus (HDV) ribozyme (SEQ ID NOs: 57, 58, 59, 60) is applied between the encoding si/shRNA sequence and the downstream termination or linearisation signal. The ribozyme sequence assures the release of correctly terminated siRNA or shRNA since the HDV ribozyme self cleaves 5' to its G + 1 nucleotide. Thus, extraneous and troublesome 3' -extra-sequences of released siRNA or shRNA are avoided by HDV ribozyme mediated cis-cleaving (Chowira et al., J. Biol. Chem. 269, 25856-64 (1994); Schürer et al., Nucleic Acids Res. 30, e56 (2002); Walker et al., Nucleic Acids Res. 31 , e82 (2003)).

### Conditional control of siRNA function in higher eukaroytic cell systems

The present invention demonstrates that the use of *N-EU-Pol* unexpectedly results in highly efficient siRNA synthesis as shown by the specific and successful gene silencing by RNA interference (see examples). The *in vivo* siRNA synthesis with *N-EU-Pol* yields high copy numbers, while omitting a cap-structure and a polyA tail. By conditionally directing the expression of a *N-Eu Pol,* it is possible to stimulate or suppress siRNA synthesis and siRNA-induced RNA interference in a cell-, organ-, or tissue-specific manner or to couple siRNA expression to specific conditions. This is achieved, by functionally linking coding sequences for a *N-EU-Pol,* preferably the DNA dependent RNA-polymerases from entero-bacteriophages T3, T7,Sp6, K11 or PhiYe03-12 (SEQ ID NOs: 1,2, 3, 43, and 44, respectively) with transcription regulatory and modifying sequences. The latter comprise promoters and enhancers or other sequences effecting transcription and gene expression. These can be chosen from prokaryotic, viral or eukaryotic sequences. Fusing the foreign RNA-polymerases, *N-EU-Pol,* with these transcription regulatory sequences leads to a directional expression of the *N-EU-Pol* corresponding to the endogenous gene taken the regulatory sequence from. This principle is exemplified by the predominant expression of the recombinant *N-EU-Pol* in hepatocytes of the liver by functional fusing the sequence coding *N-EU-Pol* with the promoter for the albumin gene (Gorski et al., *Cell,* 47, 767-76 (1986); Izban M.G. and Papaconstantinou J., *J. Biol. Chem.,* 264, 9171-9 (1989)) (cf. 5.4). Fusing the 5' flanking regulatory sequences of the cytokeratin 14 gene (SEQ I D NO: 4) with the ORF of *N EU-Pol* leads to an expression of the foreign recombinant protein in skin keratinocytes (Vassar et al., *Proc. Natl. Acad. Sci. USA,* 86, 1563-1567 (1989); Xie et al., *Cell Growth Differ.,* 9, 3132-5 (1998); Vasioukhin et al., *Proc. Natl. Acad. Sci. USA,* 96, 8551 -6 (1999)). Besides using regulatory sequences which result in tissue - or cell-type specific expression of the fused *N-EU-Pol,* one may employ sequences which mediate transcription upon stimulation or inhibition with growth factors, cytokines, physical interventions or chemical toxins or drugs. For example, the promoter of the platelet-derived growth factor B-chain, which is expressed in neurons and glial cells (Sasahara et al., *Cell,* 64, 217-27 (1991)), has been used to direct expression of human ApoE2 in the brain (Georgopoulos et al., *Biochemistry,* 41, 9293-301 (2002)). Similarly, the promoter of the intercellular adhesion molecule-2 was used to express endoglin in vascular endothelial cells (Velasco et al., *Gene Ther.,* 8, 897-904 (2001)). More sophisticated gene targeting approaches have been aiming for cell specific expression by using heterologous hypoxic and cytokine-inducible enhancers, such as the NF-kB sites of the vascular cell adhesion molecule-1 (VCAM-1) in the endothelium (Velasco et al., *Gene Ther.*,12*,* 897-904 (2001)). In hepatocytes, the promoter of the C-Reactive acute phase Protein (CRP) (SEQ I D NO: 5) directs highly enhanced expression when stimulated with acute phase mediators (Kleemann et al., *Blood,* 101, 545-51 (2003)). Here the conditional induced synthesis of an *N-EU-Pol* was demonstrated by recombinantly expressing the T7 phage polymerase gene (SEQ ID NO: 1) under the control of a CRP promoter (SEQ ID NO: 5) (see examples 5.2.2 Nummerierung anpassen) in an expression vector, when hepatoma cells transfected with this expression vector were stimulated with interleukin 1B and interleukin 6.

It is also possible to employ prokaryotic regulatory sequences to drive conditional *N-EU-Pol* expression. The tetracycline responsive elements (TRE) of the tet operator (tetO) naturally involved in prokaryotic gene expression leads to tetracycline dependent transcription, which can also be applied to the transcriptional regulation in eukaryotes. In eukaryotic cells seven co pies of tetO combined to a minimal CMV promoter (SEQ ID NO: 6) are used to control gene transcription. The available tetracycline analogue doxycycline leads to binding of the tet repressor (tet-off) or the tet activator, which is provided as a fusion protein with the VP16 protein of herpes sim plex virus (Gossen M. and Bujard H., *Proc. Natl. Acad. Sci. USA,* 89, 5547-51 (1992); Gossen M. and Bujard H., *Nucleic Acids Res.,* 21, 4411-2 (1993); Gossen et al., *Science,* 268, 1766-9 (1995)).The tet on or tet off system has already been successfully used for regulation of transgenic or recombinant expression in mammalian cells and mice (Baron U. and Bujard H., *Methods Enzymol.,* 327, 401-21 (2000); Freundlieb et al., *Methods Enzymol.,* 283, 159-73 (1997); WO 00/75347) and can be used for antibiotic controlled *N-EU-Pol* synthesis.

In all of these approaches the conditional expression of the *N-EU-Pol* results in the conditional synthesis of siRNA molecules which subsequently trigger conditional functional RNA interference in the eukaryotic cells carrying the siRNA-expression cassette presented above.

### Combination of the N-EU-Pol siRNA-expression system with the CRE / Lox system

In order to combine the *N-EU-Pol* siRNA-expression system (described above) with already existing recombinants expressing the cyclic recombinase (CRE) of the bacteriophage P1, the ORF of the *N-EU-Pol* is linked to an ubiquitous and constitutive active promoter such as the CMV promoter; in between the promoter region and the ORF of the *N-EU-Pol* a stop fragment is introduced, according to Westphal and coworker (Lakso et al., *Proc. Natl. Acad. Sci. USA,* 89, 6232-6 (1992)). The stop fragment (SEQ ID NO: 41) is flanked by loxP sites (SEQ ID NO: 42) (cf. figure 6) thereby providing Cre dependent *N-EU-Pol* expression. Crossbreeding of conditionally active Cre transgenic mice, plants or other organisms with the corresponding species carrying the recombinant *N-EU-Pol* expression cassette with IoxP stop fragment leads to broad variety of the conditional *N-EU-Pol* mediated siRNA-expresion system.

The invention is furthermore described by the following non-limiting examples.

### Examples

### Example 1: Preparation of a siRNA expression control system directed by T7-polymerase

The first major goal was the creation of a control system for a successful synthesis of functional siRNA by *N-EU-Pol.* Here, the inhibition of the synthesis of the enhanced green fluorescent protein (EGFP, SEQ ID NO: 36) mediated by the T7-RNA polym erase synthesized siRNA was chosen.

### 1 . Generation of a T7 polymerase expression cassette

First an expression cassette driving T7 RNA polymerase synthesis of the enterobacteriophage T7 gene in eukaryotic cells was developed (Fig. 2A). The coding sequence of the RNA polymerase of the enterobacteriophage T7 was PCR-amplified out from the DNA of *E. coli* strain BL21 (Tuner TM [DE3] pLacl, Novagen). Of the primers used, the forward primer (T7-RNA-P-F, SEQ ID NO: 10) comprises a 5' tail with an Nhe I restriction site, while the reverse primer (T7-RNA-P-R, SEQ ID NO: 11) has a BamH I restriction site. Alternatively, to allow monitoring of the yields of the T7-phage RNA polymerase synthesis in eukaryots, a coding sequence for T7-RNA RNA-polymerase carrying a histidine-tag at its amino terminus was generated by PCR. As primer pair, the T7-RNA-P-R (SEQ I D NO: 11) combined with the T7-RNA-P-His-F (SEQ I D NO: 12) was used. The amplification product was cloned into *pCR2.1 TOPO* (Invitrogen) thereby generating *pCR-TOPO-T7.* Thereafter, the amplification product was excised with Nhe I and BamH I and introduced into the Nhe I/BamH I cut backbone of the plasmid vector *pEGFP-C1* (BD Biosciences, Clontech). The sequence was verified by automated dideoxynucleotide sequencing using as primers the forward and reverse primer of M13 (Invitrogen), and the ones subsequently listed:

T7-F-Seq-1: CTA CTC CCT AAG ATG ATT GCA CG (SEQ ID NO: 13)

T7-F-Seq-2: ATA CGC TGA GGC TAT CGC AAC (SEQ ID NO: 14)

T7-R-Seq-3: ACT ACG CAA GGT TGG AAC ATC (SEQ ID NO: 15)

T7-F-Seq-4: ACG CTG TGT CAA TGT TCA ACC (SEQ ID NO: 16)

T7-R-Seq-5: GGA ACG GAA CCT TAT CGA CAC (SEQ ID NO: 17)

T7-R-Seq-6: TGA TTC GGC TGA GTG AAC ATC (SEQ ID NO: 18)

### 2. Establishing GFP and T7-phage RNA-Polymerase expressing cell lines

Next, stable transfectants were generated, which either expressed EGFP (SEQ I D NO: 36) or the *N-EU-Pol* of the enterobacteriophage T7, i.e the T7-phage RNA polymerase. Thus, the plasmids pEGFP C1 (BD Biosciences, Clontech), pT7-C1 or pT7-his tag-C1 were transfected either into the human hepatoma cell line Huh-7 (Nakabayashi et al., *Cancer Res.,* 42, 3858-63 (1982)) or into in the myotube differentiating cell line L6 (ATCC CRL-1458) according to the manufacturer's recommendations. Selection of stable transfectants was achieved by treating the cultures with 400 µg geneticin (Invitrogen)/ml cell culture medium for 5 days. Resistant cells were collected, and subcultured further as stably transfected cell populations, namely Huh-7-T7, L6-T7, Huh-7-GFP, and L6-GFP, by adding 200µg geneticin/ml of cell culture medium. EGFP-expression was monitored with a fluorescence microscope. The expression of the his-tagged T7-phage RNA polymerase was analysed by immunhistochemistry on paraformaldehyde-fixed cells, employing an antibody from Novagen, demonstrating cytoplasmic distribution of the T7-phage RNA polymerase.

### 3. In vivo production of GFP-interfering RNA (siRNA anti-GFP) by T7-RNA polymerase

For the expression of siRNA inhibiting the synthesis of EGFP the target sequence GGC TAG GTC CAG GAG CGC ACC (SEQ I D NO: 19) was used as published by MWG Biotech and placed under the specific control of the gene 10 promoter ofthe T7-entero-bacteriophage. The siRNA anti-GFP-expression cassette comprises the promoter sequence (T7-P, SEQ I D NO: 7) representing the binding site of the T7-phage RNA polymerase, the coding sequence for the siRNA interfering with the EGFP-transcripts (SEQ ID NO: 19), and an oligo-dT-termination sequence. The coding sequence for the siRNA against the EGFP-transcript was designed to form a small hairpin RNA (shRNA), which contains in one stem the sense directed strand, a connecting loop, and in the other stem the antisense directed strand (Fig. 3 B,C).

To insert the expression cassette into the plasm id, 5' GATC-overhangs of the restriction enzymes Bgl II and BamH I were added to the construct (cf. Fig. 3B). The expression cassette for the siRNA-antiGFP with these specifications was generated by dimerisation of two oligonucleotide-single strands. The oligonucleotides T7-GFP-S (SEQ I D NO: 20) and T7-GFP-AS (SEQ I D NO: 21) were commercially synthesized by Operon/Qiagen. Dimerization was achieved by heating them up together (96°C) in a 20 mM Tris buffer (pH 7.6) containing 150 m M NaCl, and 1 m M EDTA, followed by slow cooling in a heating block.
A pGL-3 vector (Promega), *pGL3-CMV,* was digested with Bgl II and BamH I so that the complete luciferase-expression cassette was cut out, including the CMV-promoter and the polyA-signal sequence. Ligating the oligonucleotid dimer (SEQ I D NO: 20, SEQ ID NO: 21) containing the expression cassette for siRNA-antiGFP into the remaining backbone of the pGL-3-vector, the siRNA-expression plasmid pT7-antiGFP was generated (cf fig. 2). Before transfection, the pT7-antiGFP plasmid was linearized with Nae I at its 3' terminus. The linear plasmid was then transfected in two different experiments into the cell lines Huh-T7 or Huh-GFP using Lipofectamine 2000 following manufacturer's instructions:
1 . *pT7-antiGFP + pEGFP-C1* in Huh-7 - T7
*2. pT7-antiGFP + pT7-C1* in Huh-7 - GFP
As negative controls the following experiments were conducted:
*3. pT7-antiGFP +* pBR322 in Huh-7 - GFP
4. pB-SK+¹ + *pEGFP C1* in Huh-7 ― GFP [¹ Stratagene ]
As positive control, anti-GFP siRNA oligonucleotides (MWG Biotech) were added to experiment 1 and 2 instead of pT7-antiGFP.
5. 25 nM antiGFP-siRNA (MWG Biotech) + *pEGFP C1* in Huh-7 - T7
6. 25 nM antiGFP-siRNA (MWG Biotech) + *pT7-C1* in Huh-7 - GFP GFP-expression was evaluated by light microscopy (projected in future to be done by FACS analysis). In the experiments 1 and 2, as well as in the positive control experiments 5 and 6, a reduced GFP-fluorescence was achieved. In contrast, the experiments 3 and 4 did not yield any effect on the GFP-fluorescence. Here, experiment 4 shows that the reduced GFP-expression and fluorescence is not caused artificially by co-transfection but is based on the effect of the synthesized siRNA against GFP-transcripts. Experiment 3 shows that the T7-gene 10 promoter controlling siRNA-antiGFP can only be effective, if the T7-RNA-polymerase is generated by an expression vector.
Thus for the first time, it is shown that T7-RNA-polymerase can mediate the synthesis of siRNA via the T7-RNA-polymerase recognition sequence.

### Example 2: Establishing of an eukaryotic vector system for conditional expression of siRNA

For the conditional directed siRNA synthesis two expression cassettes are needed, which can be created in one or two vectors. One of these expression cassettes comprises the promoter sequence of a *N-EU-Pol,* the siRNA coding sequence, and a terminating oligo-dT-sequence. The second one comprises the coding sequence for the *N-EU-Pol* under the transcriptionally regulatory control of an eukaryotic promoter of choice, mediating the desired conditional expression of *N-EU-Pol,* as well as a polyA signal.

### 1 . Construction of a siRNA expression vector

In order to construct the expression cassette for siRNA, restriction sites for the enzymes Eco RI, Ssp I, and Cla I were added to the 3'end of the gene 10 - promoter sequence of the bacteriophage T7. Thus, after adding a 5' GATC - overhang, the oligonucleotides T7-MCS-S (SEQ I D NO: 22) and T7-MCS-AS (SEQ ID NO: 23) (MWG Biotech) were dimerised and inserted into the BamH I and Bgl II restricted backbone of the *pGL-3-CMV* vector. Thus, two opportunities for insertion of siRNA coding oligonucleotides were created. The restriction sites EcoR I and Ssp I were used when a direction specific insertion in front of the oligo-dT had to be performed. Should an insertion of a siRNA coding sequence including the oligo-dT be desired, an EcoR I restriction digest is combined with Cla I or BamH I digestion of the siRNA cloning vector (Fig. 4).
After constructing the siRNA cloning vector, its EcoR I / Cla I cloning sites (figure 4) were used for insertion of the siRNA - anti-GFP, which was created by dimerisation of the anti-GFP-S and anti-GFP - AS (SEQ I D NO: 24 and SEQ I D NO: 25), carrying an 5' - EcoR I and a 3' Cla I restriction site. By dimerisation the dimer of anti-IGF II S / anti-IGF II AS (SEQ ID NO: 26 and SEQ ID NO: 27) using the described procedure, the anti-IGF II expression vector was created targeting the IGF I I expression.

### 2. Conditional induced synthesis of siRNA

In order to demonstrate conditional induced synthesis of siRNA mediated by a N-*EU-Pol,* we established an expression system synthesizing siRNA in hepatoma cells in response to acute phase mediators.

2.1 . Expression of T7 - RNA Polymerase in response to acute phase mediators The promoter sequence of the gene encoding the human GReactive acute phase Protein (CRP) (SEQ ID NO: 5) was amplified using human genomic DNA and the CRP primers (SEQ ID NO: 28 and SEQ I D NO: 29) which contain a Kpn I or Sac I restriction site, respectively. The amplification product was restricted with Kpn I and Sac I, and then inserted into the MCS of *pGL3 Basic vector (Promega),* resulting in *pGL-CRP-Luc.* The CRP promoter sequence was confirmed by bidirectional sequencing, and an acute phase responsive function of the promoter was confirmed by conducting luciferase reporter assays after stimulation with the acute phase mediators interleukin 1 beta (IL-1 ß) and hterleukin 6 (IL-6). For that purpose the *pGL-CRP-Luc* was transfected into Huh-7 cells. The next day, Huh-7 cells were incubated with 20 ng IL-1ß and 20 ng IL-6/ml culture media for 24 h. Luciferase reporter assays and normalisation were performed using the Dual Luciferase System of Promega according to manufacturer' s instructions.
Then, *pGL-CRP-Luc* was restricted with Nhe I and Xba I and the open reading frame (ORF) of the luciferase gene of *pGL-CRP-Luc* was replaced by the Nhe I - Xba I T7-RNA polymerase fragment of the *pCR-TOPO-T7* or the corresponding His-tagged T7-RNA polymerase of the *pCR-Topo-T7-His,* thereby generating the plasm id *pGL-CRP T7(* Fig. 5 B) *or pGL-CRP T7-His.*

### 2.2 Synthesis of siRNA in response to acute phase mediators

For siRNA mediated inhibition of gene expression in response to acute phase mediators, *pGl-CRP T7* was transiently transfected into Huh-7-GFP cells, which constitutively express the EGFP (5.1.2). Lipofectamine 2000 *(Invitrogen)* was used for transfection according to the manufacturers instructions. The BamH I-linearized *pT7 - anti-GFP* plasmid encoding for anti-GFP-siRNA was co-transfected in different ratios.
12 h after transfection, Huh-7-GFP cells were incubated with 20 ng IL-1ß and 20 ng IL-6/ml culture media resulting in a inhibition of the GFP-fluorescence.
In order to show conditional siRNA functioning on native gene expression, an anti-IGFII- siRNA construct responsive to acute phase mediators was synthesized, targeting the IGF II expression in hepatoma cells. Instead of the *pT7 anti-GFP* therefore the anti-IGFII plasmid (2.1) was co-transfected with *pGl-CRP-T7* into Huh-7 cells. Repressed m RNA level of I GF II was shown by real time PCR using the TaqMan method. Total RNA was extracted by standard procedures using commercially available kits and transcribed into cDNA by randomly primed reverse trancription using the GeneAmp RNA PCR-Kit (Applied Biosystems). For the Real Time PCR the IGF II-specific primer set (SEQ ID NOs: 30 and 31) and probe (SEQ ID NO: 32) were used for amplification in the ABI Prism 7700 (Applied Biosystems). A dilution series of total RNA confirmed linear amplification. IGF-mRNA values were normalized to the beta-actin transcript levels according to Fries et al. *Nephrol Dial Transplant* 2003, 18, 710-6) or to the 18 S rRNA levels using primers and a probe for the 18S rRNA (SEQ I D NOs: 33-35).
IGFII-mRNA levels of Huh-7 cells, in which either the *p-antil GFII* or *pGI-CRP-T7* were missing, served as negative controls. In Huh-7 cells, which were not treated with the acute phase mediators IL-1 ß and II-6, no altered IGFI I levels were detected.

### Example 3: Cell-type specific and differentiation-dependent mediated inhibition of gene expression by siRNA

Myoblasts of the rat cell line L6 differentiate into myotubes upon deprivation of serum in the culture medium. To demonstrate a differentiation-dependent siRNA function, stably transfected L6-cells, expressing the reporter EGFP under the control of the CMV promoter (L6-GFP), were chosen as expression system.
The differentiation-dependent control of siRNA synthesis was feasible via a differentiation-dependent regulation of the T7-RNA-polymerase. The procedure equalled the one described in Example 1.1. The ORF of the T7-RNA-polymerase was linked to the 3' end of a respectively functional promoter, namely the rat a-smooth-muscle actin promoter (SEQ ID NOs: 50), in the vector *pGL-3 basic.* The resulting vector construct was named *pGI-SMA-T7.*
*pGl-SMA-T7* and *pT7- anti-GFP* co-transfected Huh-7-GFP cells did not show any reduction in EGFP expression. Thus, siRNA did not show any effect in epithelial cells. An inhibition of EGFP-expression after transfection of L6-cells with recombined EGFP-expression could only be recognized due to reduced EGFP fluorescence levels once a differentiation from L6 cells into myotubes occurred following serum deprivation. The reduced expression of the reporter indicates a cell-type specific and differentiation-dependent function of siRNA, which is mediated by the directed expression of the *N-EU-Pol* T7-phage RNA polymerase.

### Example 4: Conditional siRNA delivery in diseased tissues using viral vectors

To study the conditional function of siRNA via its directed and conditionally regulated synthesis, mediated by a *N-EU-Pol, in vivo,* the siRNA expression system was introduced into mice via adenoviral gene transfer.
Hepatocellular carcinoma were induced by injection of 10⁷ Huh-7 cells into SCI D mice. Subcutaneous tumors formed after 3 to 5 weeks, which histologically resembled hepatocellular carcinoma. For tumor treatment and reduction of an IGFII expression, the anti-IGFII-siRNA expression cassette was used as described in 5.2.1
As *N-EU-Pol* the T7-phage RNA polymerase was used and placed alternatively under the control of the promoters of the CRP (SEQ ID NO: 5) or the albumin gene promoter (SEQ ID NO: 4) combined with the alpha-fetoprotein enhancer (SEQ I D NO: 51). As described before, the nucleotide sequence for CRP (cf. Example 1.1. and for the albumin-alpha-fetoprotein had been amplified from human or murine genomic DNA using adequate restriction sites and have been cloned into the vector *pGL-3 basic.* The respective Kpnl - Sal I fragment, containing the transcriptional regulatory sequence for CRP or albumin/ alpha fetoprotein, the coding region of the T7-phage RNA polymerase and the SV40-late polyA-signal of the plasmids *pGL-CRP-T7* or *pGL-Alb-T7,* were cloned into the Kpn I - Sal I restriction sites of the multiple cloning site (MCS) of the *pshuttle* vector (Stratagene). The Bgl II restriction site served as insertion site for the T7-expression cassette of the anti-IGFII - siRNA.

The plasmids pshuttle CRP-T7 or pshuttle Alb-T7 were recombined with pAdeasy 1 according to the method of He et al., *Proc. Natl. Acad. Sci. USA,* 95, 2509-14 (1998)). Subsequently, they were linearized at the Pac I site, transfected into HEK-293 cells and amplified. 5x10¹⁰ viral copies were injected intravenously into mice which had developed subcutaneous hepatocellular carcinomas. The hepatotrophic adenoviruses could be detected in tumor cells and the level of IGF II -mRNA and -protein was measured (cf. Example 2.1). Tumors which were treated with Alb-directed T7-phage RNA polymerase showed low levels of IGF II. In contrast, in tumors with CRP-directed T7-RNA RNA polymerase the IGF II - level were primarily high, and could only be reduced once IL-1 B or II-6 had been injected. This result proves that a conditional and cell-type specific siRNA function can also be achieved *in vivo* in the mammalian system.

## Claims

1. A method for the *in-vitro* modulation of the expression level of a target gene in higher eukaryotic cells or in higher eukaryotic tissue culture in a conditional manner which comprises
(a) introducing into a higher eukaryotic cell or into a higher eukaryotic tissue culture
(i) a non-eukaryotic RNA polymerase (N-EU-Pol) expression construct carrying a coding sequence for the *N-EU-Pol* lacking a nucleic signal peptide, under control of a regulating sequence providing for the conditional expression of the *N-EU-Pol,* and
(ii) a siRNA expression construct carrying one or more siRNA coding sequences under control of a recognition sequence for the *N-EU-Pol* wherein the expressed siRNA is capable of interaction with homologous mRNA sequences of the gene to be modulated expression construct; and
(b) a conditional synthesis of the *N-EU-Pol* followed by a *N-EU-Pol* driven expression of the siRNA.

2. The method of claim 1 wherein
(i) the modulation of the expression level comprises inhibition, and/or suppression of the target gene expression; and/or
(ii) the conditional manner of modulating the expression is effected by cell-, and/or tissue-, and/or development-specific factors, such as growth factors, hormones, cytokines and chemokines, and/or pathophysiological factors, such as oxygen radicals, osmotic and oxidative stress, and/or environmental factors such as temperature and ion concentration, and/or chemicals or drugs, such as antibiotics, agonists and antagonists of channel receptors, and mediators, and/or
(iii) the expressed siRNAs interact with the homologous nucleic acid sequences of the gene to be modulated; and/or
(iv) the siRNAs are expressed in the cytoplasm; and/or
(v) the siRNA- and/or the *N-EU-Pol* expression constructs are circular or linear nucleic acids, preferably the expression constructs are vectors, more preferably the expression constructs are extrachromosomal vectors, including plasmids, viruses, phages, most preferably expression constructs are viral vectors or a linear expression cassette.

3. The method of claim 1 or 2, wherein
(i) the *N-EU-Pol* lacking a nucleic signal peptide is selected from a polymerase derived from phages, viruses or bacteria preferably the *N-EU-Pol* is selected from a T7 RNA polymerase, an Sp6 RNA polymerase, a T3 RNA polymerase, a K11 RNA polymerase, a phiYeO3-12 RNA polymerase or T4 Polymerase, most preferably the *N-EU-Pol* is a T7 RNA polymerase; and/or
(ii) the regulating sequence providing for the conditional expression of the *N-EU-Pol* is a genetic element controlling the cell- and/or tissue-, and/or development-specific, and/or pathophysiological and/or environmentally induced expression of the *N-EU-Pol,* preferably the regulating sequence is selected from a cell and/or tissue and/or development-specific promoter, cell and/or tissue specific enhancers and/or from regulatory elements responsive to transcription mediating drugs, reagents, growth factors, hormones, or cytokines etc; more preferably the regulating sequence is selected from a cell-type specific promoter and/or enhancer such as the human epidermal cytokeratin 14 gene promoter, the liver-specific albumin promoter and/or enhancer, and/or the α-fetoprotein promoter and/or enhancer, or pathophysiologically responsive promoter and/or enhancers such as the liver-specific promoter of the G Reactive protein and/or enhancer, the acute phase Protein (CRP) and/or enhancer, the inflammatory inducible promoter and/or enhancer of the gene of the vascular cell adhesion molecule-1 (VCAM-1 ) or its hypoxic and/or cytokine responsive elements such as NF-?B binding sites, the promoter of the platelet derived growth factor B-chain and/or enhancer, the promoter for the intercellular adhesion molecule-2 and/or enhancer; most preferably the regulating sequence is selected fromor the tetracycline responsive elements (TRE) of the bacterial operator (tetO); and/or
(iii) the *N-EU-Pol* expression construct may comprise further genetic elements selected from ubiquitious strong promoters, recombinase recognition sites (RRS) such as lox P sites, attB/attP sites, stop codons, internal ribosome entry sites (IRES), termination signals, polyA tail, mRNA stability elements, and a hepatitis delta virus ribozyme.

4. The method according to any one of claims 1 to 3, wherein
(i) the recognition sequences for the siRNA expression construct are selected from a non-eukaryotic promoter such as bacteriophage promoters taken from the T7-, Sp6-, T3-, T4-, K11, phiYe03-12 bacteriophages etc., said recognition sequences being functionally linked with sequences encoding the siRNA, and said sequences being functionally linked with a hepatitis delta virus ribozyme sequences, these being functionally linked with specific termination signals of the promoters and/or restriction sites; and/or
(ii) the siRNAs encoded by the siRNA coding sequences of the expression construct are designated to interact with the homologous sequences of the gene to be modulated have length of 18 to 30 nucleotides, preferably of 25 to 30 nucleotides, more preferably of 18 to 25 nucleotides and/or
(iii) the siRNA expression construct comprises additional genetic elements, preferably selected from recombinase recognition sites such as lox P sites or attB/attP sites, and/or from bacterial regulatory sequences such as the tetracycline operator (tetO); and/or
(iv) the expressed siRNA forms a short hairpin RNA (shRNA) with a total length of between 41 and 85 nucleotides, with the stem being formed by the sense strand and the antisense strand and the two strands are interconnected by a loop, whereby the minimal length of the antisense and sense strands is 18 nucleotides and the maximal length is 30 nucleotides, and the loop comprises between 5 to 25 nucleotides.

5. The method of claim 3 or 4, wherein
(i) the non-eukaryotic promoter is a T7 promoter selected from SEQ ID NO: 7 and a variant thereof (SEQ ID NOs: 52, 53 and 54), an Sp6 promoter selected from SEQ ID NOs: 8, 46, 47, 48 or 49, an T3 promoter selected from SEQ I D NOs: 9 and 50, a K11 promoter selected from SEQ I D NO: 55, or a phiYe03-12 promoter selected from SEQ I D NO: 56; and/or
(ii) the termination signal for the T7 polymerase is a T7 terminator preferably having SEQ ID NOs: 37, 38, 39 or 40, the termination signal for phiYe03-12 is a phiYe03-12 terminator having SEQ I D NOs: 61 or 62 and the termination signal for the T3 polymerase is a T3 terminator preferably having SEQ I D NOs 63 or 64; and/or
(iii) the hepatitis delta virus ribozyme is selected from the group of sequences of SEQ ID NOs: 58, 59 or 60.

6. A *N-EU-Pol* expression construct as defined in any one of claims 1 to 5.

7. A siRNA expression construct as defined in any one of claims 1 to 5.

8. A kit or gene transfer agent comprising the expression constructs as defined in any one of claims 1 to 5.

9. Use of the *N-EU-Pol* expression construct as defined in any one of claims 1 to 5 for preparing a medicament for the prevention and/or treatment/therapy of viral infections, malignancies, metabolic dysfunction, chronic and acute injury, hereditary predispositions in eukaryotic organisms.

10. Use of the siRNA expression constructs as defined in any one of claims 1 to 5 for preparing a medicament for the prevention and or treatment/therapy of viral infections, malignancies, metabolic dysfunction, chronic and acute injury, hereditary predispositions in eukaryotic organisms.

11. Method for the modulation of the expression level of a target gene in higher eukaryotic cells or higher eukaryotic tissue culture o in a conditional manner which comprises introducing into an eukaryotic cell or into a cell of the tissue culture or eukaryotic organisms a *N-EU-Pol* expression construct as defined in claims 1 to 5 and a siRNA expression construct as defined in claims 1 to 5.


SEQUENCE LISTING

<110> Odenthal, Margerete
      Fries, Jochen W.U.
      Breuhahn, Kai

<120> Conditional modification of siRNAs expression

<130> 042318ep JH/BM

<140>
<141>

<150> 03020267.5
<151> 2003-09-08

<160> 70

<170> PatentIn Ver. 2.1

<210> 1
<211> 2652
<212> DNA
<213> Bacteriophage T7

<400> 1
atgaacacga ttaacatcgc taagaacgac ttctctgaca tcgaactggc tgctatcccg 60
ttcaacactc tggctgacca ttacggtgag cgtttagctc gcgaacagtt ggcccttgag 120
catgagtctt acgagatggg tgaagcacgc ttccgcaaga tgtttgagcg tcaacttaaa 180
gctggtgagg ttgcggataa cgctgccgcc aagcctctca tcactaccct actccctaag 240
atgattgcac gcatcaacga ctggtttgag gaagtgaaag ctaagcgcgg caagcgcccg 300
acagccttcc agttcctgca agaaatcaag ccggaagccg tagcgtacat caccattaag 360
accactctgg cttgcctaac cagtgctgac aatacaaccg ttcaggctgt agcaagcgca 420
atcggtcggg ccattgagga cgaggctcgc ttcggtcgta tccgtgacct tgaagctaag 480
cacttcaaga aaaacgttga ggaacaactc aacaagcgcg tagggcacgt ctacaagaaa 540
gcatttatgc aagttgtcga ggctgacatg ctctctaagg gtctactcgg tggcgaggcg 600
tggtcttcgt ggcataagga agactctatt catgtaggag tacgctgcat cgagatgctc 660
attgagtcaa ccggaatggt tagcttacac cgccaaaatg ctggcgtagt aggtcaagac 720
tctgagacta tcgaactcgc acctgaatac gctgaggcta tcgcaacccg tgcaggtgcg 780
ctggctggca tctctccgat gttccaacct tgcgtagttc ctcctaagcc gtggactggc 840
attactggtg gtggctattg ggctaacggt cgtcgtcctc tggcgctggt gcgtactcac 900
agtaagaaag cactgatgcg ctacgaagac gtttacatgc ctgaggtgta caaagcgatt 960
aacattgcgc aaaacaccgc atggaaaatc aacaagaaag tcctagcggt cgccaacgta 1020
atcaccaagt ggaagcattg tccggtcgag gacatccctg cgattgagcg tgaagaactc 1080
ccgatgaaac cggaagacat cgacatgaat cctgaggctc tcaccgcgtg gaaacgtgct 1140
gccgctgctg tgtaccgcaa ggacaaggct cgcaagtctc gccgtatcag ccttgagttc 1200
atgcttgagc aagccaataa gtttgctaac cataaggcca tctggttccc ttacaacatg 1260
gactggcgcg gtcgtgttta cgctgtgtca atgttcaacc cgcaaggtaa cgatatgacc 1320
aaaggactgc ttacgctggc gaaaggtaaa ccaatcggta aggaaggtta ctactggctg 1380
aaaatccacg gtgcaaactg tgcgggtgtc gataaggttc cgttccctga gcgcatcaag 1440
ttcattgagg aaaaccacga gaacatcatg gcttgcgcta agtctccact ggagaacact 1500
tggtgggctg agcaagattc tccgttctgc ttccttgcgt tctgctttga gtacgctggg 1560
gtacagcacc acggcctgag ctataactgc tcccttccgc tggcgtttga cgggtcttgc 1620
tctggcatcc agcacttctc cgcgatgctc cgagatgagg taggtggtcg cgcggttaac 1680
ttgcttccta gtgaaaccgt tcaggacatc tacgggattg ttgctaagaa agtcaacgag 1740
attctacaag cagacgcaat caatgggacc gataacgaag tagttaccgt gaccgatgag 1800
aacactggtg aaatctctga gaaagtcaag ctgggcacta aggcactggc tggtcaatgg 1860
ctggcttacg gtgttactcg cagtgtgact aagcgttcag tcatgacgct ggcttacggg 1920
tccaaagagt tcggcttccg tcaacaagtg ctggaagata ccattcagcc agctattgat 1980
tccggcaagg gtctgatgtt cactcagccg aatcaggctg ctggatacat ggctaagctg 2040
atttgggaat ctgtgagcgt gacggtggta gctgcggttg aagcaatgaa ctggcttaag 2100
tctgctgcta agctgctggc tgctgaggtc aaagataaga agactggaga gattcttcgc 2160
aagcgttgcg ctgtgcattg ggtaactcct gatggtttcc ctgtgtggca ggaatacaag 2220

aagcctattc agacgcgctt gaacctgatg ttcctcggtc agttccgctt acagcctacc 2280
attaacacca acaaagatag cgagattgat gcacacaaac aggagtctgg tatcgctcct 2340
aactttgtac acagccaaga cggtagccac cttcgtaaga ctgtagtgtg ggcacacgag 2400
aagtacggaa tcgaatcttt tgcactgatt cacgactcct tcggtaccat tccggctgac 2460
gctgcgaacc tgttcaaagc agtgcgcgaa actatggttg acacatatga gtcttgtgat 2520
gtactggctg atttctacga ccagttcgct gaccagttgc acgagtctca attggacaaa 2580
atgccagcac ttccggctaa aggtaacttg aacctccgtg acatcttaga gtcggacttc 2640
gcgttcgcgt aa 2652

<210> 2
<211> 2655
<212> DNA
<213> Bacteriophage T3

<400> 2
atgaacatca tcgaaaacat cgaaaagaat gacttctcag aaatcgaact ggctgctatc 60
ccgttcaaca cactggctga ccactacgga agcgccttgg ctaaagagca gttggcttta 120
gaacatgagt cttatgagct aggcgagcgc cgcttcctca agatgcttga gcgtcaagcg 180
aaagctggtg agattgcaga caacgcagcc gctaagccgt tactcgctac gcttctccct 240
aagttaacca cacgtatcgt cgagtggctc gaagagtacg catcgaagaa aggccgcaag 300
cctagcgcat acgcaccgct ccagttactc aagccggagg cctccgcgtt tatcaccctg 360
aaagttatcc ttgcgtcact aaccagtacg aacatgacaa ccattcaggc cgctgctggt 420
atgctgggga aagccattga ggacgaggca cgatttgggc gcatccgtga cctagaagcg 480
aagcacttca agaagcacgt tgaggaacag cttaacaagc gccacgggca agtctacaag 540
aaagcattta tgcaggtggt cgaggccgat atgattggtc gaggtctgct tggtggcgag 600
gcgtggtcta gctgggataa agaaaccacg atgcacgtag ggattcgcct gattgaaatg 660
ctgattgaat ccacgggtct ggtggaatta cagcgccaca acgcaggtaa cgcaggctct 720
gaccatgagg cactgcaact ggcccaagag tacgtggacg tattagcgaa gcgtgcaggc 780
gctctggcgg gtatctctcc gatgttccag ccgtgtgtcg taccgccgaa accttgggta 840
gcaatcacag ggggcggcta ttgggctaac ggtcgcagac ctttggcact cgttcgcact 900
cactctaaga agggcttgat gcgctacgaa gacgtttaca tgccagaagt ctacaaggct 960
gtgaacctcg cgcaaaacac cgcatggaaa atcaacaaga aagttcttgc tgttgtcaat 1020
gagattgtta actggaagaa ttgcccggta gcagacattc catcgctgga gcgccaagag 1080
ttaccgccta agcctgacga cattgacacc aacgaggcag cgctcaagga gtggaagaaa 1140
gccgctgctg gtatctatcg cttggacaag gcacgagtgt ctcgccgtat cagcttagag 1200
ttcatgctgg agcaggccaa caagttcgca agtaagaaag caatctggtt cccttacaac 1260
atggactggc gcggtcgtgt gtacgctgtg ccgatgttca acccgcaagg caacgacatg 1320
acgaaaggtc tgctgaccct tgctaaaggc aagccaatcg gtgaggaagg tttctactgg 1380
ctgaaaatcc acggtgcgaa ctgtgcgggt gttgataagg ttccattccc ggagcgcatc 1440
gcgttcattg agaagcacgt agacgacatt ctggcttgcg ctaaagaccc aatcaataac 1500
acttggtggg ctgagcagga ttcaccgttc tgtttcctcg cgttttgctt cgagtatgca 1560
ggcgttacgc accacggtct gagctacaat tgctctctgc cgctggcgtt cgacgggtct 1620
tgctctggta tccagcactt ctccgcgatg ctccgcgatg aggtaggcgg tcgtgcggtt 1680
aacctgctgc caagcgaaac cgtgcaggac atttacggca tcgttgcaca gaaagtaaac 1740
gagattctca aacaggatgc aatcaacggc acgcctaacg agatgattac cgtgaccgac 1800
aaggacaccg gggaaatctc agagaagctc aaacttggaa cctcaacgct ggcgcaacag 1860
tggctggcat atggtgtaac ccgtagcgta actaaacgtt cggtcatgac gctggcttac 1920
ggttccaagg agttcggctt tcgtcaacag gtattggatg acaccattca gcctgcaatt 1980
gacagcggta agggcttgat gttcacccaa ccgaaccaag cggctggcta tatggctaag 2040
ctgatttggg atgcggtaag cgtgaccgta gttgcagcgg ttgaggcgat gaactggctc 2100
aaatctgccg ctaagctgct ggctgctgag gtcaaggaca agaagaccaa ggagattctg 2160
cgccaccgtt gcgcggttca ctggactacg ccggacggct tcccggtctg gcaggaatac 2220
cgcaagccac tccagaagcg tctcgatatg attttcttag ggcaattccg tctgcaaccg 2280
acgattaata ccctcaagga ttcaggcatt gacgcacaca agcaggagtc tggcatcgct 2340
cctaactttg ttcactcaca ggacggtagc cacctccgca tgacagtcgt ttatgctcac 2400
gagaagtatg gcattgagtc ctttgcgctc atccatgaca gctttgggac tatcccggca 2460
gacgctggta agctctttaa ggctgtgcgt gaaacgatgg ttatcaccta tgagaacaac 2520
gatgtgctgg cagacttcta ctctcagttt gccgaccagc tacacgagac ccaactggac 2580
aagatgcctc cgcttccgaa gaaaggaaac ctgaacctgc aagacattct caagtctgac 2640
tttgcctttg cataa 2655

<210> 3
<211> 2625
<212> DNA
<213> Bacteriophage SP6

<400> 3
atgcaagatt tacacgctat ccagcttcaa ttagaagaag agatgtttaa tggtggcatt 60
cgtcgcttcg aagcagatca acaacgccag attgcagcag gtagcgagag cgacacagca 120
tggaaccgcc gcctgttgtc agaacttatt gcacctatgg ctgaaggcat tcaggcttat 180
aaagaagagt acgaaggtaa gaaaggtcgt gcacctcgcg cattggcttt cttacaatgt 240
gtagaaaatg aagttgcagc atacatcact atgaaagttg ttatggatat gctgaatacg 300
gatgctaccc ttcaggctat tgcaatgagt gtagcagaac gcattgaaga ccaagtgcgc 360
ttttctaagc tagaaggtca cgccgctaaa tactttgaga aggttaagaa gtcactcaag 420
gctagccgta ctaagtcata tcgtcacgct cataacgtag ctgtagttgc tgaaaaatca 480
gttgcagaaa aggacgcgga ctttgaccgt tgggaggcgt ggccaaaaga aactcaattg 540
cagattggta ctaccttgct tgaaatctta gaaggtagcg ttttctataa tggtgaacct 600
gtatttatgc gtgctatgcg cacttatggc ggaaagacta tttactactt acaaacttct 660
gaaagtgtag gccagtggat tagcgcattc aaagagcacg tagcgcaatt aagcccagct 720
tatgcccctt gcgtaatccc tcctcgtcct tggagaactc catttaatgg agggttccat 780
actgagaagg tagctagccg tatccgtctt gtaaaaggta accgtgagca tgtacgcaag 840
ttgactcaaa agcaaatgcc aaaggtttat aaggctatca acgcattaca aaatacacaa 900
tggcaaatca acaaggatgt attagcagtt attgaagaag taatccgctt agaccttggt 960
tatggtgtac cttccttcaa gccactgatt gacaaggaga acaagccagc taacccggta 1020
cctgttgaat tccaacacct gcgcggtcgt gaactgaaag agatgctatc acctgagcag 1080
tggcaacaat tcattaactg gaaaggcgaa tgcgcgcgcc tatataccgc agaaactaag 1140
cgcggttcaa agtccgccgc cgttgttcgc atggtaggac aggcccgtaa atatagcgcc 1200
tttgaatcca tttacttcgt gtacgcaatg gatagccgca gccgtgtcta tgtgcaatct 1260
agcacgctct ctccgcagtc taacgactta ggtaaggcat tactccgctt taccgaggga 1320
cgccctgtga atggcgtaga agcgcttaaa tggttctgca tcaatggtgc taacctttgg 1380
ggatgggaca agaaaacttt tgatgtgcgc gtgtctaacg tattagatga ggaattccaa 1440
gatatgtgtc gagacatcgc cgcagaccct ctcacattca cccaatgggc taaagctgat 1500
gcaccttatg aattcctcgc ttggtgcttt gagtatgctc aataccttga tttggtggat 1560
gaaggaaggg ccgacgaatt ccgcactcac ctaccagtac atcaggacgg gtcttgttca 1620
ggcattcagc actatagtgc tatgcttcgc gacgaagtag gggccaaagc tgttaacctg 1680
aaaccctccg atgcaccgca ggatatctat ggggcggtgg cgcaagtggt tatcaagaag 1740
aatgcgctat atatggatgc ggacgatgca accacgttta cttctggtag cgtcacgctg 1800
tccggtacag aactgcgagc aatggctagc gcatgggata gtattggtat tacccgtagc 1860
ttaaccaaaa agcccgtgat gaccttgcca tatggttcta ctcgcttaac ttgccgtgaa 1920
tctgtgattg attacatcgt agacttagag gaaaaagagg cgcagaaggc agtagcagaa 1980
gggcggacgg caaacaaggt acatcctttt gaagacgatc gtcaagatta cttgactccg 2040
ggcgcagctt acaactacat gacggcacta atctggcctt ctatttctga agtagttaag 2100
gcaccgatag tagctatgaa gatgatacgc cagcttgcac gctttgcagc gaaacgtaat 2160
gaaggcctga tgtacaccct gcctactggc ttcatcttag aacagaagat catggcaacc 2220
gagatgctac gcgtgcgtac ctgtctgatg ggtgatatca agatgtccct tcaggttgaa 2280
acggatatcg tagatgaagc cgctatgatg ggagcagcag cacctaattt cgtacacggt 2340
catgacgcaa gtcaccttat ccttaccgta tgtgaattgg tagacaaggg cgtaactagt 2400
atcgctgtaa tccacgactc ttttggtact catgcagaca acaccctcac tcttagagtg 2460
gcacttaaag ggcagatggt tgcaatgtat attgatggta atgcgcttca gaaactactg 2520
gaggagcatg aagtgcgctg gatggttgat acaggtatcg aagtacctga gcaaggggag 2580
ttcgacctta acgaaatcat ggattctgaa tacgtatttg cctaa 2625

<210> 4
<211> 2376
<212> DNA
<213> Artificial Sequence

<220>
<223> Description of Artificial Sequence: Albumin
      promoter region of Mus musculus (clone pHH3-1);
      accession No: J04738

<400> 4
   aagcttgaaa acaggactgc cttagaagta actaataatt atgtggctca tcctctagta 60
   tagagtctac atataggacg agtgcccagg agtttcagaa ggaagagtat aaattcttac 120
   tcaccagatg caaaagtgtt tctcttcagt tgaaatgatc tgtgagcacg gagactgtgt 180
   caagaattaa gcactttaat tctcggctgc taaagattaa ttagaacatg cccgagtaag 240
   gcattgtgtt agtactgcct aaggagaggc tcagttgtta ttaatttatg tcttaaaagt 300
   attggagctt catcagtcac taaaccagct caagcgccta cttcattctg atgcttttct 360
   ctccaggctt tggctgcata gatgattttt tttttttttt ttacttaatt gtcaaacaaa 420
   agtatatata gtatacatag tacatgtgag agtgctaaaa gcctgagtta ataacacaat 480
   attatactta aacacaatat tacatataac tatatagtac ttgtgagtta ataatacaaa 540
   tacattcaat gacttaagca ttacatactt gtttatgttt atcaagaccc acaccaggac 600
   aggactaggc atgactactg tcttcaaata aatagtgact actctttcta ggaagtttaa 660
   agaggaattg atccaagaaa gaattatctc ttctgtttag tttctttttt tcttttccag 720
   gatggggttt tggcttaatt ctgccaccca ccctcaccct cagtagaagt tattccatgg 780
   gcagacttta tctatctatc tatctatcta tctatctatc tatctatcta tctatctatc 840
   tatctaccta cctacctccc tatccatcta tcatctatct atctatctat ctatctatct 900
   atctatctat ctatcatcta tctatctatc tatcattctg ttcatctgtg tagaggctag 960
   tctgttattc catttcagga ctcttttgag tgataaaaca agcaaggaaa cacttttccc 1020
   ttctccttca ttttgccttg actcctgtgg ttgattacag aatttgtaca cctttctgaa 1080
   gatgaggaca caagatgagg tcataagaca tagatttctt tcattgtata accagatata 1140
   aagaagaatg cttctcctcc tcctcctctt cctcctcctc ctctcttcct ccttttcctt 1200
   cttctcctcc ttcctcttct ccttcttgtc actgaatagt ccctgtatat ttaggaacca 1260
   tggggttgat ttggatgtag tcttgactga ctcataagtg agataactgg tctgctacta 1320
   attattatcc tgtggtgtta gaggaagtat gttacgatgg caaagaccat catcactgtc 1380
   attggagtgc ctgttctagt gtctcctcta aagcaccccg agaaagaggt tcatgttaga 1440
   tgaaaacatt ctactccaga cccgtttcac acatagcctg agcatggggt ggggacacaa 1500
   gacttctgaa agtcctctgc acttgttttt tattgctctg gcttcttctc agttcacatt 1560
   tgagacactg tgtgctttat ggaagattct aactttaaaa agatgagaaa gacttggaag 1620
   ccctataatg agacccaccc ctccctcata cacattcttc attacctgta cataaaagca 1680
   tatttgggat gaacaaccta tgcaattcag ttcttagctt ttaaatgtgg catgcttcca 1740
   tgccaaggcc cacactgaaa tgctcaaatg ggagacaaag agattaagct cttatgtaaa 1800
   atttgctgtt ttacataact ttaatgaatg gacaaagtct tgtgcatggg ggtgggggtg 1860
   gggttagagg ggaacagctc cagatggcaa acatacgcaa gggatttagt caaacaactt 1920
   tttggcaaag atggtatgat tttgtaatgg ggtaggaacc aatgaaatgc gaggtaagta 1980
   tggttaatga tctacagtta ttggttaaag aagtatatta gagcgagtct ttctgcacac 2040
   agatcacctt tcctatcaac cccactagcc tctggcaaaa tgaagtgggt aacctttctc 2100
   ctcctcctct tcgtctccgg ctctgctttt tccaggggtg tgtttcgccg agaagcacgt 2160
   aagagtttta tgttttttca tctctgcttg tatttttcta gtaatggaag cctggtattt 2220
   taaaatagtt aaattttcct ttagtgctga tttctagatt attattactg ttgttgttgt 2280
   tattattgtc attatttgca tctgagaacc cttaggtggt tatattattg atatattttt 2340
   ggtatctttg atgacaataa tggggatttg aagctt 2376

<210> 5
<211> 301
<212> DNA
<213> Artificial Sequence

<220>
<223> Description of Artificial Sequence: Promoter
      region 1425 - 1726 of the human C-reactive
      protein, pentraxin-related (CRP) gene; accession
      No.: AF449713

<400> 5
acccagatgg ccactcgttt aatatgttac catttcccat tattttcgca ggatagatag 60
ccaaagtgga gccctgagag atttcttcat ttttcctgtc ataaagaatt ggtaattcag 120
tagtcatagg agtttgtaat aaataactca cattgatttc tctgttctga aataattttg 180
cttcccctct tcccgaagct ctgacacctg ccccaacaag caatgttgga aaattattta 240
catagtggcg caaactccct tactgctttg gatataaatc caggcaggag gaggtagctc 300
t 301

<210> 6

<211> 444
<212> DNA
<213> Artificial Sequence

<220>
<223> Description of Artificial Sequence: Pmin-CMV + TRE
      7x (tetO operators)

<400> 6
ctcgagttta ccactcccta tcagtgatag agaaaagtga aagtcgagtt taccactccc 60
tatcagtgat agagaaaagt gaaagtcgag tttaccactc cctatcagtg atagagaaaa 120
gtgaaagtcg agtttaccac tccctatcag tgatagagaa aagtgaaagt cgagtttacc 180
actccctatc agtgatagag aaaagtgaaa gtcgagttta ccactcccta tcagtgatag 240
agaaaagtga aagtcgagtt taccactccc tatcagtgat agagaaaagt gaaagtcgag 300
ctcggtaccc gggtcgagta ggcgtgtacg gtgggaggcc tatataagca gagctcgttt 360
agtgaaccgt cagatcgcct ggagacgcca tccacgctgt tttgacctcc atagaagaca 420
ccgggaccga tccagcctcc gcgg                                         444


<210> 7
<211> 18
<212> DNA
<213> Artificial Sequence

<220>
<223> Description of Artificial Sequence: T7-promoter
      gene 10

<400> 7
ttaatacgac tcactata                                                18


<210> 8
<211> 25
<212> DNA
<213> Artificial Sequence

<220>
<223> Description of Artificial Sequence: Sp6 promoter
      of the Sp6 RNA polymerase gene

<400> 8
atttaggtgg acactataga aggag                                        25


<210> 9
<211> 23
<212> DNA
<213> Artificial Sequence

<220>
<223> Description of Artificial Sequence: T3-RNA
      polymerase promoter

<400> 9
aattaaccct cactaaaggg aga                                          23


<210> 10
<211> 43
<212> DNA
<213> Artificial Sequence

<220>
<223> Description of Artificial Sequence: Primer
      T7-RNA-P-F

<400> 10
ctacgctagc gtcgccacca tgaacacgat taacatcgct aag                43


<210> 11
<211> 34
<212> DNA
<213> Artificial Sequence

<220>
<223> Description of Artificial Sequence: Primer
      T7-RNA-P-R

<400> 11
attcggatcc ttacgcgaac gcgaagtccg actc                          34


<210> 12
<211> 68
<212> DNA
<213> Artificial Sequence

<220>
<223> Description of Artificial Sequence: PRIMER
      T7-RNA-P-His-F

<400> 12
gctacgctag ccggtcgcca ccatgcatca ccatcaccat cataacacga ttaacatcgc 60
taagaacg                                                       68


<210> 13
<211> 23
<212> DNA
<213> Artificial Sequence

<220>
<223> Description of Artificial Sequence: Primer
      T7-F-Seq-1

<400> 13
ctactcccta agatgattgc acg                                      23


<210> 14
<211> 21
<212> DNA
<213> Artificial Sequence

<220>
<223> Description of Artificial Sequence: Primer
      T7-F-Seq-2

<400> 14
atacgctgag gctatcgcaa c                                        21


<210> 15
<211> 21

<212> DNA
<213> Artificial Sequence

<220>
<223> Description of Artificial Sequence: Primer
      T7-R-Seq-3

<400> 15
actacgcaag gttggaacat c                                                21


<210> 16
<211> 21
<212> DNA
<213> Artificial Sequence

<220>
<223> Description of Artificial Sequence: Primer
      T7-F-Seq-4

<400> 16
acgctgtgtc aatgttcaac c                                                21


<210> 17
<211> 21
<212> DNA
<213> Artificial Sequence

<220>
<223> Description of Artificial Sequence: Primer
      T7-R-Seq-5

<400> 17
ggaacggaac cttatcgaca c                                                21


<210> 18
<211> 21
<212> DNA
<213> Artificial Sequence

<220>
<223> Description of Artificial Sequence: Primer
      T7-R-Seq-6

<400> 18
tgattcggct gagtgaacat c                                                21


<210> 19
<211> 21
<212> DNA
<213> Artificial Sequence

<220>
<223> Description of Artificial Sequence: GFP target
      sequence

<400> 19
ggctacgtcc aggagcgcac c                                                21

<210> 20
<211> 78
<212> DNA
<213> Artificial Sequence

<220>
<223> Description of Artificial Sequence:
      oligonucleotide T7-GFP-S

<400> 20
gatcttaata cgactcacta tagggctacg tccaggagcg caccttcaag agaggtgcgc 60
tcctggacgt agccggcg                                                78


<210> 21
<211> 78
<212> DNA
<213> Artificial Sequence

<220>
<223> Description of Artificial Sequence:
      oligonucleotide T7-GFP-AS

<400> 21
gatccgccgg ctacgtccag gagcgcacct ctcttgaagg tgcgctcctg gacgtagccc 60
tatagtgagt cgtattaa                                                78


<210> 22
<211> 49
<212> DNA
<213> Artificial Sequence

<220>
<223> Description of Artificial Sequence:
      oligonucleotide T7-MCS-S

<400> 22
gatcttaata cgactcacta tagaattcaa gagaatattt ttatcgatg              49


<210> 23
<211> 49
<212> DNA
<213> Artificial Sequence

<220>
<223> Description of Artificial Sequence:
      Oligonucleotide T7-MSC-As

<400> 23
aattatgctg agtgatatct taagttctct tataaaaata gctacctag              49


<210> 24
<211> 59
<212> DNA
<213> Artificial Sequence

<220>
<223> Description of Artificial Sequence:
      oligonucleotide antiGFP-S

<400> 24
aattcggcta cgtccaggag cgcaccttca agagaggtgc gctcctggac gtagccaat  59


<210> 25
<211> 55
<212> DNA
<213> Artificial Sequence

<220>
<223> Description of Artificial Sequence:
      oligonucleotide antiGFP-AS

<400> 25
attggctacg tccaggagcg cacctctctt gaaggtgcgc tcctggacgt agccg      55


<210> 26
<211> 54
<212> DNA
<213> Artificial Sequence

<220>
<223> Description of Artificial Sequence:
      oligonucleotide IGF II-S

<400> 26
aattctatga cacctggaag cagtttcaag agaactgctt ccaggtgtca taat       54


<210> 27
<211> 50
<212> DNA
<213> Artificial Sequence

<220>
<223> Description of Artificial Sequence:
      oligonucleotide IGF II-AS

<400> 27
attatgacac ctggaagcag ttctcttgaa actgcttcca ggtgtcatag            50


<210> 28
<211> 40
<212> DNA
<213> Artificial Sequence

<220>
<223> Description of Artificial Sequence: CRP primer F
      (Kpn I)

<400> 28
ccgggtacca cccagatggc cactcgttta atatgttacc                       40


<210> 29
<211> 31
<212> DNA
<213> Artificial Sequence

<220>
<223> Description of Artificial Sequence: CRP primer R

(Sac I)

<400> 29
cctgagctca gagctacctc ctcctgcctg g                                    31


<210> 30
<211> 20
<212> DNA
<213> Artificial Sequence

<220>
<223> Description of Artificial Sequence: IGF primer-1

<400> 30
gtgcttctca ccttcttggc                                                 20


<210> 31
<211> 20
<212> DNA
<213> Artificial Sequence

<220>
<223> Description of Artificial Sequence: IGF primer-2

<400> 31
gtcttgggtg ggtagagcaa                                                 20


<210> 32
<211> 23
<212> DNA
<213> Artificial Sequence

<220>
<223> Description of Artificial Sequence: IGF II probe

<400> 32
ttcagggagg ccaaacgtca ccg                                             23


<210> 33
<211> 20
<212> DNA
<213> Artificial Sequence

<220>
<223> Description of Artificial Sequence: 18S rRNA
      primer-1

<400> 33
aaacggctac cacatccaag                                                 20


<210> 34
<211> 20
<212> DNA
<213> Artificial Sequence

<220>
<223> Description of Artificial Sequence: 18S rRNA
      primer-2

<400> 34
cctccaatgg atcctcgtta                                                   20


<210> 35
<211> 20
<212> DNA
<213> Artificial Sequence

<220>
<223> Description of Artificial Sequence: 18S rRNA probe

<400> 35
agcaggcgcg caaattaccc                                                   20


<210> 36
<211> 798
<212> DNA
<213> Artificial Sequence

<220>
<223> Description of Artificial Sequence: EGFP

<400> 36
atggtgagca agggcgagga gctgttcacc ggggtggtgc ccatcctggt cgagctggac 60
ggcgacgtaa acggccacaa gttcagcgtg tccggcgagg gcgagggcga tgccacctac 120
ggcaagctga ccctgaagtt catctgcacc accggcaagc tgcccgtgcc ctggcccacc 180
ctcgtgacca ccctgaccta cggcgtgcag tgcttcagcc gctaccccga ccacatgaag 240
cagcacgact tcttcaagtc cgccatgccc gaaggctacg tccaggagcg caccatcttc 300
ttcaaggacg acggcaacta caagacccgc gccgaggtga agttcgaggg cgacaccctg 360
gtgaaccgca tcgagctgaa gggcatcgac ttcaaggagg acggcaacat cctggggcac 420
aagctggagt acaactacaa cagccacaac gtctatatca tggccgacaa gcagaagaac 480
ggcatcaagg tgaacttcaa gatccgccac aacatcgagg acggcagcgt gcagctcgcc 540
gaccactacc agcagaacac ccccatcggc gacggccccg tgctgctgcc cgacaaccac 600
tacctgagca cccagtccgc cctgagcaaa gaccccaacg agaagcgcga tcacatggtc 660
ctgctggagt tcgtgaccgc cgccgggatc actctcggca tggacgagct gtacaagtcc 720
ggactcagat ctcgagctca agcttcgaat tctgcagtcg acggtaccgc gggcccggga 780
tccaccggat ctagataa                                                     798


<210> 37
<211> 24
<212> DNA
<213> Artificial Sequence

<220>
<223> Description of Artificial Sequence: T7-
      transcription terminator

<400> 37
gttatgctag ttattgctca gcgg                                              24


<210> 38
<211> 36
<212> DNA
<213> Artificial Sequence

<220>
<223> Description of Artificial Sequence:
      T7-transcription terminator

<400> 38
aaccccttgg ggcctctaaa cgggtcttga ggggtt                                36

<210> 39
<211> 28
<212> DNA
<213> Artificial Sequence

<220>
<223> Description of Artificial Sequence: T7
      transcription terminator

<400> 39
ggggtttttt gctgaaagga ggaactat                                         28

<210> 40
<211> 35
<212> DNA
<213> Artificial Sequence

<220>
<223> Description of Artificial Sequence:
      T7-transcription terminator

<400> 40
cgtttataag gagacacttt atgtttaaga aggtt                                 35

<210> 41
<211> 1578
<212> DNA
<213> Artificial Sequence

<220>
<223> Description of Artificial Sequence: Westphal stop
      fragment (Not I fragment pBS 302 GIBCO Life
      Technologies)

<400> 41
gcggccgcac gtctaagaaa ccattattat catgacatta acctataaaa ataggcgtat 60
cacgaggccc tttcgtcttc aagaattcca tcaagcttag gatccggaac ccttaatata 120
acttcgtata atgtatgcta tacgaagtta ttaggtccct cgacctgcag cccaagctta 180
cttaccatgt cagatccaga catgataaga tacattgatg agtttggaca aaccacaact 240
agaatgcagt gaaaaaaatg ctttatttgt gaaatttgtg atgctattgc tttatttgta 300
accattataa gctgcaataa acaagttaac aacaacaatt gcattcattt tatgtttcag 360
gttcaggggg aggtgtggga ggttttttaa agcaagtaaa acctctacaa atgtggtatg 420
gctgattatg atctctagtc aaggcactat acatcaaata ttccttatta acccctttac 480
aaattaaaaa gctaaaggta cacaattttt gagcatagtt attaatagca gacactctat 540
gcctgtgtgg agtaagaaaa aacagtatgt tatgattata actgttatgc ctacttataa 600
aggttacaga atatttttcc ataattttct tgtatagcag tgcagctttt tcctttgtgg 660
tgtaaatagc aaagcaagca agagttctat tactaaacac agcatgactc aaaaaactta 720
gcaattctga aggaaagtcc ttggggtctt ctacctttct cttctttttt ggaggagtag 780
aatgttgaga gtcagcagta gcctcatcat cactagatgg catttcttct gagcaaaaca 840
ggttttcctc attaaaggca ttccaccact gctcccattc atcagttcca taggttggaa 900
tctaaaatac acaaacaatt agaatcagta gtttaacaca ttatacactt aaaaatttta 960
tatttacctt agagctttaa atctctgtag gtagtttgtc caattatgtc acaccacaga 1020
agtaaggttc cttcacaaag atccctcgag aaaaaaaata taaaagagat ggaggaacgg 1080
gaaaaagtta gttgtggtga taggtggcaa gtggtattcc gtaagaacaa caagaaaagc 1140
atttcatatt atggctgaac tgagcgaaca agtgcaaaat ttaagcatca acgacaacaa 1200
cgagaatggt tatgttcctc ctcacttaag aggaaaacca agaagtgcca gaaataacat 1260

gagcaactac aataacaaca acggcggcta caacggtggc cgtggcggtg gcagcttctt 1320
tagcaacaac cgtcgtggtg gttacggcaa cggtggtttc ttcggtggaa acaacggtgg 1380
cagcagatct aacggccgtt ctggtggtag atggatcgat ggcaaacatg tcccagctcc 1440
aagaaacgaa aaggccgaga tcgccatatt tggtgtcccc gaggatccgg aacccttaat 1500
ataacttcgt ataatgtatg ctatacgaag ttattaggtc cctcgaagag gttcactagt 1560
actggccatt gcggccgc 1578

<210> 42
<211> 34
<212> DNA
<213> Artificial Sequence

<220>
<223> Description of Artificial Sequence: flanking loxP
      sequences

<400> 42
ataacttcgt atagcataca ttatacgaag ttat 34

<210> 43
<211> 2843
<212> DNA
<213> Bacteriophage K11

<400> 43
acgaggtgta tcttaggtgt ctccgaacgg tgaggcaccc atagataaac tttatccaca 60
aagaggcaca caatgaacgc attaaacatt ggacgtaatg acttctccga gattgaactt 120
gctgctattc cgtacaacat cctgagcgag cactacgggg accaagcggc acgtgagcag 180
ttagcactgg agcatgaggc atacgagctt ggcagacaac gtttcctgaa gatgttagaa 240
cgtcaggtga aagctggtga gttcgctgac aacgcggccg ctaagccgct ggtcttaacg 300
ttgcacccac agctgaccaa gcgcattgac gactggaagg aggagcaagc aaacgctcgc 360
ggtaagaagc ctcgcgcata ctacccgatt aagcacggcg tcgcctcaga gttagctgtt 420
agcatgggcg ctgaggtgct aaaagagaag cgcggagtgt ccagtgaggc aatcgcactg 480
ctgaccatta aggtcgtctt ggggaacgct cacagaccgc tcaaaggcca caatccagca 540
gtatcctctc agttaggcaa ggctcttgag gatgaggccc gcttcggtcg tatccgtgag 600
caggaagccg catacttcaa gaagaacgta gcggaccagc tggacaagcg agtaggccac 660
gtgtacaaga aggctttcat gcaggtagtc gaggccgata tgatatccaa agggatgctg 720
ggcggcgaca actgggcgag ctggaaaact gacgagcaga tgcacgtagg gaccaagctg 780
ctggagctac tcattgaggg aactggtctg gtggaaatga ccaagaacaa gatggccgat 840
ggctccgatg atgtaaccag tatgcagatg gtccagctgg ctccggcctt tgtggaactc 900
ctgagcaaac gggcaggcgc actcgcgggt atcagcccga tgcaccagcc gtgcgtagtc 960
cctccgaaac cttgggtgga gaccgtaggc ggtggctact ggtcagtcgg tcgccgtccg 1020
ctggcactgg tgcgtaccca ctccaagaag gcgctgcgcc gctacgctga cgtgcacatg 1080
ccagaggtat acaaggcggt aaacctcgcg caaaacacgc cgtggaaggt gaacaagaag 1140
gtgctggcgg tagtcaacga gattgtcaac tggaagcact gcccggtagg tgacgtccca 1200
gcgattgaac gcgaagagtt accgccgcgc ccggacgata ttgacaccaa cgaggtggca 1260
cgtaaggcat ggcgcaagga ggccgcagcg gtctaccgta aggacaaggc ccgccagtct 1320
cgccgttgtc gatgtgagtt catggtcgca caggctaaca agttcgctaa ccacaaggcc 1380
atttggttcc cgtacaacat ggactggcgc gggcgtgtgt acgctgtgag catgttcaac 1440
ccacagggta acgatatgac caaggggagt ctgacgctgg ccaagggtaa gccaattggt 1500
ctcgacgggt tctactggct gaagattcac ggcgcaaact gtgcaggtgt cgacaaggtt 1560
cccttccctg agcgcatcaa gttcatcgaa gagaacgagg gcaacattct ggcgagcgca 1620
gcggacccgc tgaataacac ttggtggacc cagcaagatt cgccgttctg tttcttagcg 1680
ttctgcttcg agtacgcagg tgttaagcat cacggcctga attacaactg ctcgctgccg 1740
ctggcgttcg atgggtcctg ctctgggatt cagcacttca gcgcgatgct ccgagattcc 1800
atcggtggtc gtgcggttaa cctgctgcct tctgataccg tgcaggatat ctacaagatt 1860
gtggccgaca aggtgaacga agtgctccac cagcacgccg tcaacgggtc tcagaccgtg 1920
gtcgagcaga ttgctgacaa agagactggc gagtttcacg agaaggtgac tctgggcgag 1980
tccgtactgg ctgcgcagtg gttgcaatat ggtgtgaccc gcaaggtgac taagcgttcg 2040
gtcatgacgc tggcatacgg ttccaaagag tcgttggtcc gccagcaggt tcttgaggac 2100
accattcagc ctgctattga caacggcgag ggcctgatgt ttacgcaccc taaccaagca 2160

gctggctaca tggctaagct gatttgggac gctgtgaccg tgaccgtagt ggccgctgtc 2220
gaggcaatga actggctgaa gtctgccgct aagctgctgg ctgctgaagt caaggacaag 2280
aagaccaaag aggtgctgcg taagcgctgc gcaatccact gggtaacacc cgatggcttc 2340
ccggtgtggc aggagtaccg caagcagaac caagcgcgcc tgaagctggt cttcctcggg 2400
caggccaacg tcaagatgac gtataacact gggaaggact ccgagattga tgcccacaag 2460
caggaatccg gcatcgctcc taactttgtt cactcacagg atggcagtca cctgcgcatg 2520
actgtagtac acgccaacga ggtctacggg attgactcct tcgcactcat tcacgactcc 2580
tctgggacca ttccggctga cgctgggaat ctctttaagg cagtccgcga gacgatggtc 2640
aagacctacg aggacaacga tgtaattgca gacttctacg accagtttgc cgaccagctg 2700
cacgagtctc aactggacaa gatgcctgcg gttccggcca aaggtgacct gaatctgcgc 2760
gatatcttgg agtctgactt cgcgtttgcg taaggtctca ggcaattagg gcacactata 2820
gggaaccttc gaatgaccga ggg 2843

<210> 44
<211> 2655
<212> DNA
<213> Bacteriophage PL-1

<400> 44
atgaacatca tcgaaaacat cgaaaagaat gacttctccg aaatcgaact ggctgctatc 60
ccgttcaaca cactggctga ccactacggc agtgcattgg ctcgtgaaca actggcttta 120
gagcatgagt cttatgagct aggcgagcgc cgcttcctca agatgcttga gcgtcaagcg 180
aaagctggtg agattgcaga caacgcagcc gctaagccat tactcgctac gctccttcct 240
aagctaacca cacgcatcgt tgagtggcta gaagagtacg ccacgaagaa aggccgcaag 300
cctgtagcct atgcaccact ccaatcactc aagccggagg cgtccgcgtt tatcacactc 360
aaggtcatcc ttgcgtcact gaccagcacg aacatgacaa ccattcaggc cgctgctggt 420
atgcttggga aggccattga ggatgaggca cgtttcgggc gcatccgtga cctcgaagcg 480
aagcacttca agaagcacgt tgaggaacag cttaacaagc gccacgggca agtctacaag 540
aaagccttta tgcaggtggt tgaggcagat atgattggtc gcgggctgct gggaggtgag 600
gcgtggtcta gctgggacaa ggaaaccacg atgcacgtag ggattcgcct gattgaaatg 660
ctgattgaat ccacgggtct ggtggaatta cagcgccaca atgcaggtaa cgcaggctct 720
gaccatgagg cactgcaact ggcccaagag tacgtggacg tattagcgaa gcgtgcaggc 780
gctctggcgg gtatctctcc gatgttccag ccgtgtgtcg tcccgccgaa accttgggta 840
gcaatcaccg ggggcggcta ttgggctaac ggtcgcagac ctttggcact cgttcgcact 900
cactctaaga agggactgat gcgctatgag gatgtttaca tgccagaagt ttacaaggcg 960
gtcaacatcg cacagaacac cgcatggaaa atcaacaaga aagttcttgc tgttgtcaat 1020
gagattgtta actggaagaa ttgcccggtc gcagacattc catcgctgga gcgccaagag 1080
ttaccgccta agccggacga cattgacacc aacgaggcag cactcaagga gtggaagaaa 1140
gccgccgctg gtatataccg cttggacaag gcacgagtgt ctcgacgtat cagcttagag 1200
ttcatgctgg agcaggccaa caagttcgca agcaagaaag caatctggtt cccttacaac 1260
atggactggc gcggtcgtgt gtacgctgtg ccgatgttca acccgcaagg caacgacatg 1320
acgaaaggtc tgctgaccct cgctaaaggc aagccaatcg gtgaggaagg gttctactgg 1380
ctgaaaatcc acggtgcgaa ctgtgcgggt gtcgataagg ttcctttccc tgagcgcatc 1440
gcgttcattg agaagcacgt agacgacatt ctggcttgcg ctaaagaccc aatcaataac 1500
acttggtggg ccgagcagga ttcacctttc tgtttcctag cgttctgctt cgagtatgca 1560
ggcgttgcgc accacggtct gagctacaat tgctccctgc cgctggcgtt cgacgggtct 1620
tgctctggta tccagcactt ctccgcgatg ctacgcgatg aggtaggcgg tcgtgcggtt 1680
aacctgctgc caagcgaaac cgtgcaggac atttacggca tcgttgcgca gaaagtaaac 1740
gagattctca aacaggatgc aatcaacggc acacctaacg agatgattac cgtgaccgat 1800
aaggacaccg gggaaatctc tgagaagctc aagctgggaa cctcaacgct ggcgcaacag 1860
tggctggcat atggtgtaac ccgtagcgta actaaacgtt cggtcatgac gctggcttac 1920
ggttccaagg agttcggctt tcgtcaacag gtactggacg acaccattca gcctgcgatt 1980
gacagcggta agggcttgat gttcacccaa cctaaccaag cggctggcta tatggctaag 2040
ctgatttggg acgcggtgag cgtgaccgta gtcgcagcgg ttgaggcgat gaactggctc 2100
aagtctgccg ctaagctgct ggctgctgag gtcaaggaca agaagaccaa ggagattctg 2160
cgccaccgtt gcgcggttca ctggactaca ccggacggct tcccggtctg gcaggaatac 2220
cgcaagccac tacagaagcg tctcgatatg attttcttag ggcaattccg tctgcaaccg 2280
acgattaata ccctcaagga ttcaggcatt gacgcacaca agcaggagtc tggcatcgcg 2340
cccaactttg ttcactcaca ggatggtagc cacctccgca tgacagtcgt ttatgctcac 2400
gagaattatg gcattgagtc ctttgcgctc atccatgaca gctttgggac tatcccggca 2460
gacgctggta agctctttaa ggctgtgcgt gaaacgatgg ttatcaccta tgagaacaac 2520
gatgtgctgg cagacttcta tgaccagttt gcagaccagc tacacgagac ccaactggac 2580

aagatgcctc ctcttccgaa gaaagggaac ctgaacctgc aagacattct caagtctgac 2640
tttgcctttg cataa 2655

<210> 45
<211> 2350
<212> DNA
<213> Artificial Sequence

<220>
<223> Description of Artificial Sequence: Promoter
      region of the human epidermal keratin 14 (KRT14)
      gene; accession No.: U11076

<400> 45
aagcttatat tccatgctag ggttctggtg ttggtgcgtg gggttggggt gggactgcag 60
aagtgccttt taagattatg tgattgactg atctgtcatt ggttccctgc catctttatc 120
ttttggattc ccctcggagg aggggaggaa ggagtttctt ttgggtttta ttgaatcaaa 180
tgaaagggaa agtagaggtg ttcctatgga ggggaggaag gagtttcttt tgggttttat 240
tgaatcaaat gaaagggaaa gtagaggtgt tcctatgtcc cgggctccgg agcttctatt 300
cctgggccct gcataagaag gagacatggt ggtggtggtg gtgggtgggg gtggtggggc 360
acagaggaag ccgatgctgg gctctgcacc ccattcccgc tcccagatcc ctctggatat 420
agcaccccct ccagtgagca cagcctcccc ttgccccaca gccaacagca acatgcctcc 480
caacaaagca tctgtccctc agccaaaacc cctgttgcct ctctctgggg aaattgtagg 540
gctgggccag ggtgggggga ccattctctg cagggagatt aggagtgtct gtcaggggcg 600
ggtggagcgg ggtggggccc tggcttactc acatccttga gagtcctttg ctggcagatt 660
tggggagccc acagctcaga tgtctgtctc agcattgtct tccaagctcc taggccacag 720
tagtggggcg ctcccttctc tggcttcttc tttggtgaca gtcaaggtgg ggttgggggt 780
gacgaagggt cctgcttctc ttctaggagc agttgatccc aggaagagca ttggagcctc 840
cagcaggggc tgttggggcc tgtctgagga gataggatgc gtcaggcagc cccagacacg 900
atcacattcc tctcaacatg cctgccgggg tctgtggagc cgaggggctg atgggagggt 960
ggggtggggg ccggaagggt ttgctttggg aggttgtctg ggagattgct gaagttttga 1020
tatacacacc tccaaagcag gaccaagtgg actcctagaa atgtcccctg acccttgggg 1080
cttcaggagt cagggaccct cgtgtccacc tcagccttgc ccttgcacag cccagctcca 1140
ctccagcctc tactcctccc cagaacatct cctgggccag ttccacaagg ggctcaaacg 1200
agggcacctg agctgcccac actagggatg ttctgggggt ctgagaagat atctggggct 1260
ggaagaataa aaggcccccc taggcctgtt cctggatgca gctccagcca ctttggggct 1320
aagcctgggc aataacaatg ccaacgaggc ttcttgccat actcggttta caaaaccctt 1380
tacatacatt gtcgcattgg attctcagag ctgactgcac taagcagaat agatggtatg 1440
actcccactt tgcagatgag aacactgagg ctcagagaag tgcgaagccc tgggtcacag 1500
aggcgtaaat gcagagccag gacccacctg aagacccacc tgactccagg atgtttcctg 1560
cctccatgag gccacctgcc ctatggtgtg gtggatgtga gatcctcacc ataggaggga 1620
gattagggtc tgtgctcagg gctggggaga ggtgcctgga tttctctttg atggggatgt 1680
tggggtggga atcacgatac acctgatcag ctgggtgtat ttcagggatg gggcagactt 1740
ctcagcacag cacggcaggt caggcctggg agggcccccc agacctcctt gtctctaata 1800
gagggtcatg gtgagggagg cctgtctgtg cccaaggtga ccttgccatg ccggtgcttt 1860
ccagccgggt atccatcccc tgcagcagca ggcttcctct acgtggatgt taaaggccca 1920
ttcagttcat ggagagctag caggaaacta ggtttaaggt gcagaggccc tgctctctgt 1980
caccctggct aagcccagtg cgtgggttcc tgagggctgg gactcccagg gtccgatggg 2040
aaagtgtagc ctgcaggccc acacctcccc ctgtgaatca cgcctggcgg gacaagaaag 2100
cccaaaacac tccaaacaat gagtttccag taaaatatga cagacatgat gaggcggatg 2160
agaggaggga cctgcctggg agttggcgct agcctgtggg tgatgaaagc caaggggaat 2220
ggaaagtgcc agacccgccc cctacccatg agtataaagc actcgcatcc ctttgcaatt 2280
tacccgagca ccttctcttc actcagcctt ctgctcgctc gctcacctcc ctcctctgca 2340
ccatgactac 2350

<210> 46
<211> 63
<212> DNA
<213> Artificial Sequence

<220>

<223> Description of Artificial Sequence: Sp6 promoter,
      accession No.: M31342

<400> 46
ctcgcggtct ttaattgcct atttaggtga cactatagaa gggaggtagt cctccctaac 60
cta                                                               63


<210> 47
<211> 63
<212> DNA
<213> Artificial Sequence

<220>
<223> Description of Artificial Sequence: Sp6 promoter;
      accession No.; M31343

<400> 47
aagaatttca aggacttggt aattagggga cactatagaa ggaggccgag gaataacagg 60
aag                                                               63


<210> 48
<211> 63
<212> DNA
<213> Artificial Sequence

<220>
<223> Description of Artificial Sequence: Sp6 promoter
      of the pGEM vector; accession No.: M31344

<400> 48
tggacaaatc tgtgtctctt atttagggga cactatagaa gagagaattt taatcggcga 60
tag                                                               63


<210> 49
<211> 20
<212> DNA
<213> Artificial Sequence

<220>
<223> Description of Artificial Sequence: T3 promoter

<400> 49
atttaggtga cactatagaa                                             20


<210> 50
<211> 743
<212> DNA
<213> Artificial Sequence

<220>
<223> Description of Artificial Sequence: promoter of
      the alpha smooth muscle actin gene (region -713 -
      +52)

<400> 50
gcggtcctta agcatgatat caagggtcag cgataaacca acaacatgca cgtggactgt 60
acctaagggt taacgcagtt acagtgattc tgacttctaa gttcctctta gggtaacata 120
ggctggtgaa tcctgattac atacttccat atgtaataca tacagacttc attgatacta 180
cacacagact ccagactaca tacaatgtgg cttccataaa atgatcactc ctctgcagat 240

tcgcaggtga cccaagcatc ttttgttata ggctaccttt tgcaacagtg ttgccttaaa 300
gtcccagcta gtcagagaca ggcccttcct catctcaagc ccttagctaa tggacccaaa 360
ggctagcctg acaggaagag ctggcatctt ctgaggaatg tgcaaaccat gcctgcgtct 420
gctccatggc actagcccag tgtctgggca tttgagcagt tgttctgagg gctcaggatg 480
tttatcccca taagcagctg aactgcctcc tgtttcgaga gcagagcaga ggaatgcagt 540
ggaagagacc caggcctctg gcacccagat tagagagttt tgtgctgagg tccctatatg 600
gttgtgttag agtgaacggc cagcttcagc ctgtctttgc tccttgtttg ggaagcgagt 660
gggaggggat cagaccaggg ggctatataa cccttcagca ttcagcctcc ccagacacca 720
cccacccaga gtggagaagc ccc 743

<210> 51
<211> 300
<212> DNA
<213> Artificial Sequence

<220>
<223> Description of Artificial Sequence:
      alpha-fetoprotein enhancer 1 of Mus musculus

<400> 51
ataaagctgg tttgagtttt tgtcatctgg ctgacagttt gaatagtcaa gggtagagcc 60
tttcatagac taagtccagt ttgtttaacc atattgcttc tgagaggttt catgggctca 120
ggaagtgaca acagaaccag acatagagct atgagaggta tgtggtgtga gctcagtatt 180
tgcgacaaag ctttgagcaa cagcacacgt gggcttaggg ttgtttgcag ttggtattag 240
agacctcaca caaagctatg tggcagataa cggtgtgggc aaaattcaaa cccagtcgcc 300

<210> 52
<211> 20
<212> DNA
<213> Artificial Sequence

<220>
<223> Description of Artificial Sequence: T7 promoter
      variant

<400> 52
taatacgtct cactataggg 20

<210> 53
<211> 20
<212> DNA
<213> Artificial Sequence

<220>
<223> Description of Artificial Sequence: T7 promoter
      variant

<400> 53
taataggact cactataggg 20

<210> 54
<211> 23
<212> DNA
<213> Artificial Sequence

<220>
<223> Description of Artificial Sequence: T7 promoter
      variant

<400> 54
caatacgact cactataggg aga                                              23


<210> 55
<211> 23
<212> DNA
<213> Artificial Sequence

<220>
<223> Description of Artificial Sequence: K12 promoter

<400> 55
aattagggca cactataggg aac                                              23


<210> 56
<211> 23
<212> DNA
<213> Artificial Sequence

<220>
<223> Description of Artificial Sequence: phiYeO3-12
      promoter phi1.05

<400> 56
cattaaccct cactaacggg ata                                              23


<210> 57
<211> 72
<212> DNA
<213> Artificial Sequence

<220>
<223> Description of Artificial Sequence: hepatitis
      delta virus (HDV) ribozyme

<400> 57
ggccggcatg gtcccagcct cctcgctggc gccggctggg caacaccatt gcactccggt 60
ggcgaatggg ac                                                          72


<210> 58
<211> 67
<212> DNA
<213> Artificial Sequence

<220>
<223> Description of Artificial Sequence: hepatitis
      delta virus (HDV) ribozyme

<400> 58
gggtcggcat ggcatctcca cctcctcgcg gtccgacctg ggctacttcg gtaggctaag 60
ggagaag                                                                67


<210> 59
<211> 70
<212> DNA
<213> Artificial Sequence

<220>

<223> Description of Artificial Sequence:
      self-processing hepatitis delta virus ribozyme

<400> 59
gggtcggcat ggcatctcca cctcctcgcg gtccgacctg ggctacttcg gtaggctaag 60
ggagaagctt                                                         70


<210> 60
<211> 37
<212> DNA
<213> Artificial Sequence

<220>
<223> Description of Artificial Sequence: hepatitis
      delta virus (HDV) ribozyme deletion variant

<400> 60
ggtcggcatg gcatctccac ctcctcgcgg tcagctt                           37


<210> 61
<211> 16
<212> DNA
<213> Artificial Sequence

<220>
<223> Description of Artificial Sequence: Tphi
      terminator aus YeO3-12

<400> 61
ggctccttcg ggagtc                                                  16


<210> 62
<211> 21
<212> DNA
<213> Artificial Sequence

<220>
<223> Description of Artificial Sequence: Tphi
      terminator aus YeO3-12

<400> 62
cctctttggg agtctgaggg g                                            21


<210> 63
<211> 22
<212> DNA
<213> Artificial Sequence

<220>
<223> Description of Artificial Sequence: T3 terminator
      TE

<400> 63
ggctcacctt cacgggtggg cc                                           22


<210> 64
<211> 39
<212> DNA

<213> Artificial Sequence

<220>
<223> Description of Artificial Sequence: T3 terminator
      Tphi

<400> 64
aaaccccttg ggttccctct ttgggagtct gaggggttt                          39


<210> 65
<211> 44
<212> DNA
<213> Artificial Sequence

<220>
<223> Description of Artificial Sequence: rrnB T1
      transcriptional terminator for Sp6 polymerase

<400> 65
ataaaacgaa aggcccagtc ttccgactga gcctttcgtt ttat                    44


<210> 66
<211> 28
<212> DNA
<213> Artificial Sequence

<220>
<223> Description of Artificial Sequence: rrnB T2
      transcriptional  terminator for Sp6 polymerase

<400> 66
aaaaggccat ccgtcaggat ggccttct                                      28


<210> 67
<211> 3317
<212> DNA
<213> Artificial Sequence

<220>
<223> Description of Artificial Sequence: T7 polymerase
      expression cassette including a promoter, a T7 pol
      gene and a polyA tail

<400> 67
cacccagatg gccactcgtt taatatgtta ccatttccca ttattttcgc aggatagata 60
gccaaagtgg agccctgaga gatttcttca tttttcctgt cataaagaat tggtaattca 120
gtagtcatag gagtttgtaa taaataactc acattgattt ctctgttctg aaataatttt 180
gcttcccctc ttcccgaagc tctgacacct gccccaacaa gcaatgttgg aaaattattt 240
acatagtggc gcaaactccc ttactgcttt ggatataaat ccaggcagga ggaggtagct 300
ctgagctctt acgcgtgcta gccggtcgcc accatgaaca cgattaacat cgctaagaac 360
gacttctctg acatcgaact ggctgctatc ccgttcaaca ctctggctga ccattacggt 420
gagcgtttag ctcgcgaaca gttggccctt gagcatgagt cttacgagat gggtgaagca 480
cgcttccgca agatgtttga gcgtcaactt aaagctggtg aggttgcgga taacgctgcc 540
gccaagcctc tcatcactac cctactccct aagatgattg cacgcatcaa cgactggttt 600
gaggaagtga aagctaagcg cggcaagcgc ccgacagcct tccagttcct gcaagaaatc 660
aagccggaag ccgtagcgta catcaccatt aagaccactc tggcttgcct aaccagtgct 720
gacaatacaa ccgttcaggc tgtagcaagc gcaatcggtc gggccattga ggacgaggct 780
cgcttcggtc gtatccgtga ccttgaagct aagcacttca agaaaaacgt tgaggaacaa 840
ctcaacaagc gcgtagggca cgtctacaag aaagcattta tgcaagttgt cgaggctgac 900
atgctctcta agggtctact cggtggcgag gcgtggtctt cgtggcataa ggaagactct 960

attcatgtag gagtacgctg catcgagatg ctcattgagt caaccggaat ggttagctta 1020
caccgccaaa atgctggcgt agtaggtcaa gactctgaga ctatcgaact cgcacctgaa 1080
tacgctgagg ctatcgcaac ccgtgcaggt gcgctggctg gcatctctcc gatgttccaa 1140
ccttgcgtag ttcctcctaa gccgtggact ggcattactg gtggtggcta ttgggctaac 1200
ggtcgtcgtc ctctggcgct ggtgcgtact cacagtaaga aagcactgat gcgctacgaa 1260
gacgtttaca tgcctgaggt gtacaaagcg attaacattg cgcaaaacac cgcatggaaa 1320
atcaacaaga aagtcctagc ggtcgccaac gtaatcacca agtggaagca ttgtccggtc 1380
gaggacatcc ctgcgattga gcgtgaagaa ctcccgatga aaccggaaga catcgacatg 1440
aatcctgagg ctctcaccgc gtggaaacgt gctgccgctg ctgtgtaccg caaggacaag 1500
gctcgcaagt ctcgccgtat cagccttgag ttcatgcttg agcaagccaa taagtttgct 1560
aaccataagg ccatctggtt cccttacaac atggactggc gcggtcgtgt ttacgctgtg 1620
tcaatgttca acccgcaagg taacgatatg accaaaggac tgcttacgct ggcgaaaggt 1680
aaaccaatcg gtaaggaagg ttactactgg ctgaaaatcc acggtgcaaa ctgtgcgggt 1740
gtcgataagg ttccgttccc tgagcgcatc aagttcattg aggaaaacca cgagaacatc 1800
atggcttgcg ctaagtctcc actggagaac acttggtggg ctgagcaaga ttctccgttc 1860
tgcttccttg cgttctgctt tgagtacgct ggggtacagc accacggcct gagctataac 1920
tgctcccttc cgctggcgtt tgacgggtct tgctctggca tccagcactt ctccgcgatg 1980
ctccgagatg aggtaggtgg tcgcgcggtt aacttgcttc ctagtgaaac cgttcaggac 2040
atctacggga ttgttgctaa gaaagtcaac gagattctac aagcagacgc aatcaatggg 2100
accgataacg aagtagttac cgtgaccgat gagaacactg gtgaaatctc tgagaaagtc 2160
aagctgggca ctaaggcact ggctggtcaa tggctggctt acggtgttac tcgcagtgtg 2220
actaagcgtt cagtcatgac gctggcttac gggtccaaag agttcggctt ccgtcaacaa 2280
gtgctggaag ataccattca gccagctatt gattccggca agggtctgat gttcactcag 2340
ccgaatcagg ctgctggata catggctaag ctgatttggg aatctgtgag cgtgacggtg 2400
gtagctgcgg ttgaagcaat gaactggctt aagtctgctg ctaagctgct ggctgctgag 2460
gtcaaagata agaagactgg agagattctt cgcaagcgtt gcgctgtgca ttgggtaact 2520
cctgatggtt tccctgtgtg gcaggaatac aagaagccta ttcagacgcg cttgaacctg 2580
atgttcctcg gtcagttccg cttacagcct accattaaca ccaacaaaga tagcgagatt 2640
gatgcacaca aacaggagtc tggtatcgct cctaactttg tacacagcca agacggtagc 2700
caccttcgta agactgtagt gtgggcacac gagaagtacg gaatcgaatc ttttgcactg 2760
attcacgact ccttcggtac cattccggct gacgctgcga acctgttcaa agcagtgcgc 2820
gaaactatgg ttgacacata tgagtcttgt gatgtactgg ctgatttcta cgaccagttc 2880
gctgaccagt tgcacgagtc tcaattggac aaaatgccag cacttccggc taaaggtaac 2940
ttgaacctcc gtgacatctt agagtcggac ttcgcgttcg cgtaaggatc cgaatttaag 3000
ggcgaattct gcagatatcc atcacactgg cggccgctcg agcatgcatc tagagtcggg 3060
gcggccggcc gcttcgagca gacatgataa gatacattga tgagtttgga caaaccacaa 3120
ctagaatgca gtgaaaaaaa tgctttattt gtgaaatttg tgatgctatt gctttatttg 3180
taaccattat aagctgcaat aaacaagtta acaacaacaa ttgcattcat tttatgtttc 3240
aggttcaggg ggaggtgtgg gaggtttttt aaagcaagta aaacctctac aaatgtggta 3300
aaatcgataa ggatccg 3317

<210> 68
<211> 108
<212> DNA
<213> Artificial Sequence

<220>
<223> Description of Artificial Sequence:
      siRNA-expression construct comprising a T7
      promoter, an shRNA directed against IGF(shRNA
      anti-IGF), and an  HDV ribozyme variant

<400> 68
ttaatacgac tcactatagg gtatgacacc tggaagcagt ttcaagagaa ctgcttccag 60
gtgtcatatt gggtcggcat ggcatctcca cctcctcgcg gtcagctt 108

<210> 69
<211> 70
<212> DNA
<213> Artificial Sequence

<220>
<223> Description of Artificial Sequence: dimerisation
      partner of SEQ ID NO 70; dimerised SEQ ID 69 and
      SEQ ID NO 70 form a double-stranded expression
      construct

<400> 69
ttaatacgac tcactatagg gtatgacacc tggaagcagt ttcaagagaa ctgcttccag 60
gtgtcatatt                                                         70


<210> 70
<211> 70
<212> DNA
<213> Artificial Sequence

<220>
<223> Description of Artificial Sequence: dimerisation
      partner of SEQ ID NO 69; dimerised SEQ ID 70 and
      SEQ ID NO 69 form a double-stranded expression
      construct

<400> 70
aatatgacac ctggaagcag ttctcttgaa actgcttcca ggtgtcatac cctatagtga 60
gtcgtattaa                                                         70